# EUROPEAN PATENT SPECIFICATION

(11) **EP 1 187 938 B1**
(45) Date of publication and mention of the grant of the patent: **09.11.2011**
(21) Application number: 00941769.2
(22) Date of filing: 01.06.2000
(51) Int. Cl.: C12N 15/85, A01K 67/033, C12N 15/90

(54) **METHOD OF REGULATED EXPRESSION OF TRANSGENES IN THE GERMLINE OF CAENORHABDITIS ELEGANS**
METHODE ZUR EXPRESSION VON TRANSGENE IN DER KEIMBAHN VON CAENORHABDITIS ELEGANS
PROCEDE D'EXPRESSION DE TRANSGENES DANS LA LIGNEE GERMINALE DE CAENORHABDITIS ELEGANS

(30) Priority: 01.06.1999 US 136972 P
(43) Date of publication of application: 20.03.2002
(60) Divisional application: 10012765.3
(73) Proprietor: The University of Utah Research Foundation, Salt Lake City, UT 84108 (US); INSTITUT NATIONAL DE LA SANTE ET DE LA RECHERCHE MEDICALE (INSERM), 75654 Paris Cédex 13 (FR)
(72) Inventor: Bessereau, Jean-Louis, 46, rue D'Ulm, F-75005 Paris (FR); Jorgensen, Erik, Salt Lake City, UT 84102 (US)
(74) Representative: Pawlyn, Anthony Neil
(86) International application number: PCT/US2000/040091
(87) International publication number: WO 2000/073510

(56) References cited:
- US-A- 6 051 430
- FIRE A ET AL: "A modular set of lacZ fusion vectors for studying gene expression in Caenorhabditis elegans" GENE, ELSEVIER, AMSTERDAM, NL, vol. 93, no. 2, 14 September 1990 (1990-09-14), pages 189-198, XP002002218 ISSN: 0378-1119
- FUKUSHIGE T. ET AL.: 'Modulation of gene expression in the embryonic digestive tract of C. elegans' DEVELOPMENTAL BIOLOGY vol. 178, 1996, pages 276 - 288
- VAN LUENEN H. ET AL.: 'The mechanism of transposition of Tc3 in C. elegans' CELL vol. 79, 21 October 1994, pages 293 - 301
- VOS J. ET AL.: 'Transposase is the only nematode protein required for in vitro transposition of Tc1' GENES & DEVELOPMENT vol. 10, 1996, pages 755 - 761
- PLASTERK R.: 'Reverse genetics of caenorhabditis elegans' BIOESSAYS vol. 14, no. 9, September 1992, pages 629 - 633
- PLASTERK R. ET AL.: 'Targeted alterations of the caenorhabditis elegans genome by transgene instructed DNA double strand break repair following Tc1 excision' THE EMBO JOURNAL vol. 11, no. 1, 1992, pages 287 - 290
- GRUIDL M. ET AL.: 'Multiple potential germ-line helicases are components of the germ-line-specific P granules of caenorhabditis elegans' PROC. NATL. ACAD. SCI. USA vol. 93, November 1996, pages 13837 - 13842

## Description

### CROSS-REFERENCE TO RELATED APPLICATION

This application is a national stage entry of and claims priority under 35 U.S.C. § 365 to PCT/US00/40091, filed June 1, 2000, designating the United States of America, corresponding to PCT International Publication WO 00/073510 (published in English on December 7, 2000), which claims the benefit of U.S. Provisional Application No. 60/136,972, filed June 1, 1999.

### 1. FIELD OF THE INVENTION

The present disclosure relates to methods for generating and identifying mutations in the genome of the nematode *Caenorhabditis elegans* (hereinafter "*C. elegans"*). More specifically, the present invention relates to a method of regulated expression of a transgene in cells of the germline of *C. elegans*, also disclosed are methods for regulating mobilization of heterologous or endogenous transposons in the *C. elegans* genome, inserting a heterologous DNA sequence into *C. elegans* gennline DNA, and engineering mutations into the *C. elegans* genome.

### 2. TECHNICAL BACKGROUND

The use of model genetic systems had its beginnings in the earliest days of the science of genetics and, as a result of the tremendous value of such systems in understanding genetic phenomena, continues in the present. Researchers often use in their work organisms which have short life spans, limited space requirements, and relatively small genomes. Specifically, certain species of worms, fruit flies, and yeast cells are common subjects of research. Using such organisms, researchers may learn the function of the various genes found within the DNA of the organisms. One commonly used method is to generate mutations in the genome of an organism, followed by selection or screening for those mutations which confer a specific property or characteristic to the organism. These mutational studies suggest probable functions for the genes in which mutations occur. Mutations often occur when a gene is changed in such a way that the product of the gene is altered or nonfunctional.

A common method for generating mutations uses transposable elements. Transposable elements are segments of DNA which have the ability to "hop"-that is, to be excised from their initial position in the DNA and move to a new location. In doing this, a transposable element, also known as a transposon, may insert into some portion of a gene, thus disrupting or even changing the function of the gene. Further, additional mutations may be created by remobilizing the transposon. Since this remobilization often occurs imperfectly, changes are created in the DNA sequence, leaving the final sequence different from the original sequence. *See* J.D. Watson, J. Witkowski, M. Gilman, and M. Zoller, Recombinant DNA 175-190, 439-440 2d. ed. (1996).

The P element, a transposable element found in the genes of fruit flies, *see, e.g.,* A. C. Spradling, G. M. Rubin, Science 218, 341 (1982); J.D. Watson, J. Witkowski, M. Gilman, and M. Zoller, Recombinant DNA 175, 177 2d. ed. (1996), has been an enormously useful tool in *Drosophila* genetic analysis for two reasons. First, these transposons have been used for insertional mutagenesis. Mutagenic insertions constitute molecular tags that are used to rapidly clone the mutated gene. L. Cooley, R. Kelley, A. Spradling, Science 239, 112 (1988). Particularly helpful in such studies is the presence of strains that lack any copies of the transposon. Second, P elements are used to introduce single copies of foreign sequences into the host genome. This feature is particularly useful for the rapid identification of gene expression patterns by using enhancer traps. H. J. Bellen, et al., Genes Dev. 3, 1288 (1989). The availability of such techniques would be particularly advantageous in studies of the genome of the nematode *C. elegans.*

*C. elegans* is a model system in which genetics can be used to identify genes and biological pathways which are conserved between nematodes and vertebrates, and which thus constitute potential targets for the treatment of various diseases. *C. elegans* is particularly advantageous for genetic studies because it is easily propagated and because the genetic and physical maps of its genome are well-characterized. W. B. Wood, Introduction to C. elegans Biology (1988). The characterization of gene structure in *C. elegans* has become routine, largely through the efforts of the *C. elegans* genome project. The workers involved in this effort have cloned the entire genome into cosmid or YAC vectors and have completed the genomic sequence. C. elegans Sequencing Consortium, Science 282:2012-2018 (1998); A. Coulson et al., Proc. Natl. Acad. Sci. USA 83:7821-7825 (1986); A. Coulson et al., Bioessays 13:413-417 (1991); R. Wilson et al., Nature 368:32-38 (1994).

Standard mutagenesis in *C. elegans* employs chemical mutagens. After generation of a mutant, identification of the gene requires time-consuming genetic mapping followed by single gene rescue. Alternatively, transposon-based mutagenesis has been attempted using mutant backgrounds like *mut-*2, but efficiency of transposition is low and not specific for a defined transposon class. Further, since the genomes of all *C. elegans* strains contain transposons, it is very difficult to identify relevant insertions. Thus, utility of native transposons for regulated transposition in *C. elegans* is limited. First, all strains contain multiple copies of these transposons and thus new insertions do not provide unique tags. Second, mutator strains tend to activate the transposition of several classes of transposons, so that the type of transposon associated with a particular mutation is not known. Third, transposition is not regulated and the transposon tag can be lost by excision in subsequent generations. Fourth, attempts to regulate transposase expression have failed because expression of transgenes in the germline of *C. elegans* is very difficult. Although one could theoretically regulate the transposition of a specific element by expressing the transposase under the control of a germline-specific promoter, transgenic arrays are typically silenced in the gennline. W. G. Kelly, S. Xu, M. K. Montgomery, A. Fire, Genetics 146, 227 (1997).

Another problem in this field is the difficulty of expressing DNA in the C. *elegans* gennline. Current methods, *see, e.g.,* W. G. Kelly et al., Genetics 146:227-238 (1997), are not adequate. First, current methods for expressing foreign DNA in the *C. elegans* germline do not work for all genes. Second, expression of genes introduced using these methods declines over time.

Finally, introduction of single copy DNA is not possible using existing technology.

From the foregoing, it will be appreciated that it would be a significant advancement in the art to provide methods that allow regulated expression of foreign DNA in the *C. elegans* germline. It would be a further advancement to provide methods that allow gennline expression of a transgene in *C. elegans.* It would be a further advancement in the art to provide regulated expression of such a transgene in the germline, as by regulation using a heat-shock promoter. It would be a further advancement to provide methods of regulating the transposition of either endogenous or heterologous transposons in *C. elegans.* Further, it would be an advancement to provide transgene constructs to facilitate germline expression of transgenes and regulated transposition of homologous and heterologous transposons. Such compositions of matter and methods are disclosed herein.

### 3. BRIEF SUMMARY OF THE INVENTION

Herein disclosed are improved methods for generating and identifying mutations in *C. elegans,* the invention relates to methods for introducing heterologous DNA into the *C. elegans* germline and causing its expression. In certain embodiments, a method of the present invention comprises the steps of inserting a transgene construct into the *C*. *elegans,* wherein the construct comprises a heterologous gene operably linked to a promoter and a 3' untranslated region of a gene that is expressed in the *C. elegans* germline; and expressing the heterologous gene. In certain embodiments, this method further comprises the removal of all bacterial plasmid sequences and repeated sequences from the DNA to be introduced. In certain preferred embodiments, a promoter that is active in the *C. elegans* germline drives expression of the transgene. In certain especially preferred embodiments, the promoter is an inducible promoter.

Also disclosed is a transgene construct for expression in *C. elegans* which comprises a heterologous gene operably linked to a promoter and a 3' untranslated region of a gene expressed in the *C. elegans* genome. In certain embodiments, the transgene construct further comprises a promoter that is active in the germline of *C. elegans* or a promoter that is inducible.

Herein disclosed are also methods for generating and identifying mutations in *C. elegans.* In one embodiment, a method herein disclosed comprises the introduction and expression of a transposase gene to mobilize either endogenous or heterologous transposons. In certain preferred embodiments, the transposons are endogenous Tc3 transposons.

In certain other embodiments, the transposons are heterologous transposons, such as the *Drosophila* mariner element. Controlled mobilization of heterologous transposons allows the generation of mutations, which are tagged by the insertion of the transposon. PCR-based techniques permit rapid identification of the transposon insertion that caused the mutation.

Also disclosed herein are methods for introducing single copy DNA sequences into *C. elegans.* In certain preferred embodiments, a method herein disclosed comprises introducing a transposon comprising a heterologous DNA sequence into a *C. elegans,* introducing a transgene construct comprising a transposase gene operably linked to a promoter and a 3' untranslated region of a gene that is expressed in the *C. elegans* germline, and expressing the transposase such that the transposase integrates into a *C. elegans* chromosome as a single copy. The transposon may be engineered to introduce a DNA sequence, such as one that codes for a reporter gene such as, for example, a green fluorescent protein. The introduced DNA sequence may also contain FRT/FLP or CRE/LOX recombination sites. Alternatively, the introduced DNA sequence may contain polyadenylation sites or transcriptional terminators.

These and other features and advantages of the present invention will become more fully apparent from the following detailed description.

### 4. SUMMARY OF THE DRAWINGS

Figure 1 schematically depicts a method for mutagenesis by controlled heterologous transposition.
Figure 2 depicts the structure of the pJL44 *Mos1* transposase expression vector.
Figure 3 schematically depicts a method for identifying sequences flanking the *Mos1* insertion site using inverse PCR.
Figure 4 depicts the sequence (SEQ ID NO: 23) of an inverse PCR product. Nucleotides in capital letters are from the *Mos1* transposon. The *C. elegans*-flanking genomic region is in lower case. It matches the Y47C4.A sequence from chromosome X available at the Sanger Centre. *See* the Sanger Centre web site at http://www.sanger.ac.uk/Projects/C elegans/.
Figure 5 depicts the mobilization of *Mos1* in C. elegance somatic cells. (A) Engineering of the *Mos* transposase encoding sequence.
   Restriction sites were generated at the 5'and 3' ends of the coding sequence (new sequence is indicated under the original sequence). The endogenous polyadenylation signal (boxed) was disrupted and an artificial intron (SEQ ID NO: 18) was introduced in the coding sequence in order to improve transposase expression. *See* A. Fire, S. W. Harrison, D. Dixon, Gene 93,189 (1990). (B) Localization of *Mos 1* insertions in *unc-49* and *gpa-2* genes after induction of *Mos* transposase expression in somatic cells. Open triangles: insertion sites; black rectangles: coding exons; white rectangle: non coding exonic sequence. Arrows: genomic primers used to amplify the insertions. (C) Sequence comparison of 22 insertion sites. Insertion sites are oriented relative to the 5' end of the *Mos 1* transposon. Sequences that flank *Mos 1* at the right end were identified by PCR. DNA purification and PCR were performed as described in H. G. van Luenen, S. D. Colloms, R. H. Plasterk, Embo J. 12, 2513 (1993). The primers in *Mos 1* were oJL88 (5'-CGCATGCGGCTTACTCAC (SEQ ID NO: 4)) first PCR; and oJL89 (5'-GGCCCCATCCGATTACCACCTA (SEQ ID NO: 5)) second PCR. Primers in *unc-49* were oJL19 (5'-GCGAAACGCATACCAACTGTA (SEQ ID NO: 6)) first PCR; and oJL20 (5'-TTCATGCCGAAAAGCAGGCGT (SEQ ID NO: 7)) second PCR. Primers in *gpa*-2 were the same as described in H. G. van Luenen, S. D. Colloms, R. H. Plasterk, Embo J. 12, 2513 (1993). PCR products were gel-purified and sequenced using oJL89 (SEQ ID NO: 5) as a primer. (positive positions on the graph), sequences that flank the left end *of Mos 1* were deduced from *unc-49* and *gpa-2* sequences (negative positions on the graph).

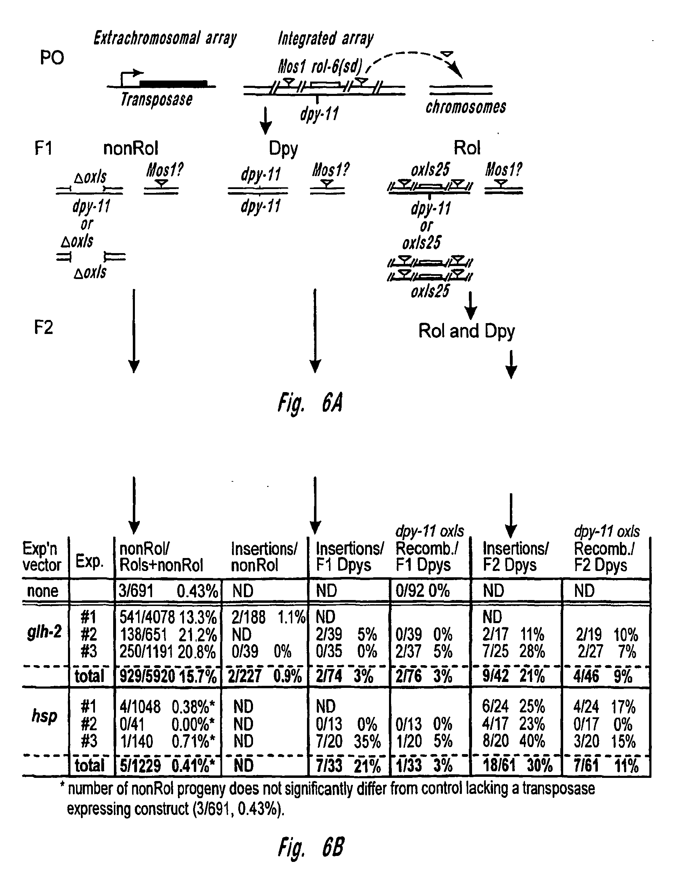

Figure 6 depicts germline mobilization of *Mos 1.* (A) *Mos* transposase was expressed from an extrachromosomal array using either a *glh-2* or a heat-shock promoter. The *Mos* 1 transposon was contained in an array integrated on chromosome V (*oxls25 [Mos 1; rol-6 (sd]).* The array containing chromosome was balanced by the dpy-11 (e224) mutation. In the next generation, catastrophic excision of the transgene was observed (indicated as ΔoxIs) among the progeny. (B) Comparison of excision and insertion frequencies using *glh-2* and heat-shock (hsp) promoters to drive *Mos* transposase expression in the germline. New *Mos l* insertions were identified by PCR. Specifically, the presence of *Mos l* was detected through PCR by using two primers located in the transposon, oJL102 (SEQ ID NO: 1) and 1JL103 SEQ ID NO: 3). The absence *of D. mauritiana*-flanking sequence was checked using oJL102 (SEQ ID NO: 1) and oJL104 (SEQ ID NO: 2) as described below. In addition, a PCR positive control was performed on each DNA sample using oligonucleotides located in the *cha-1* gene. Recombination events were recognized as Dpy worms also containing *Mos1* flanked by original Drosophila genomic sequences.

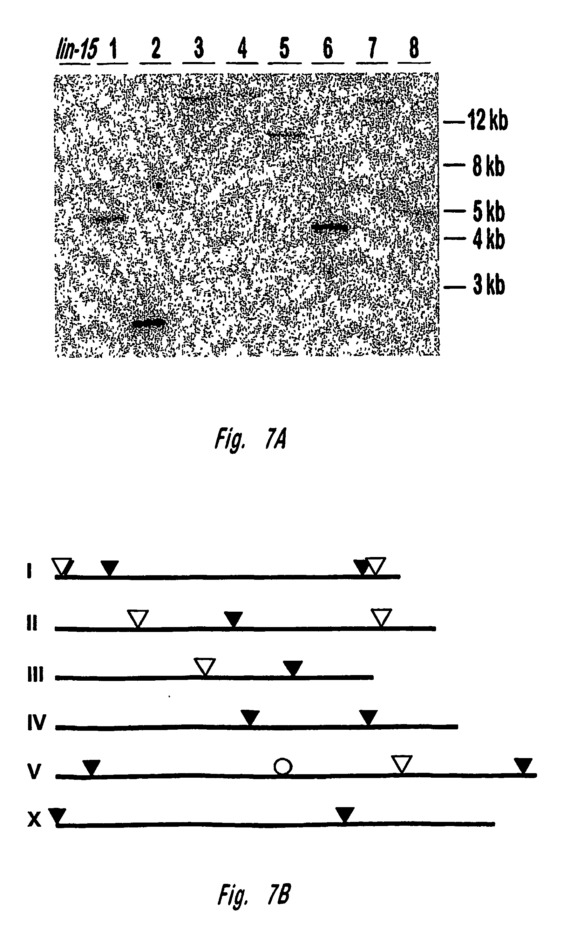

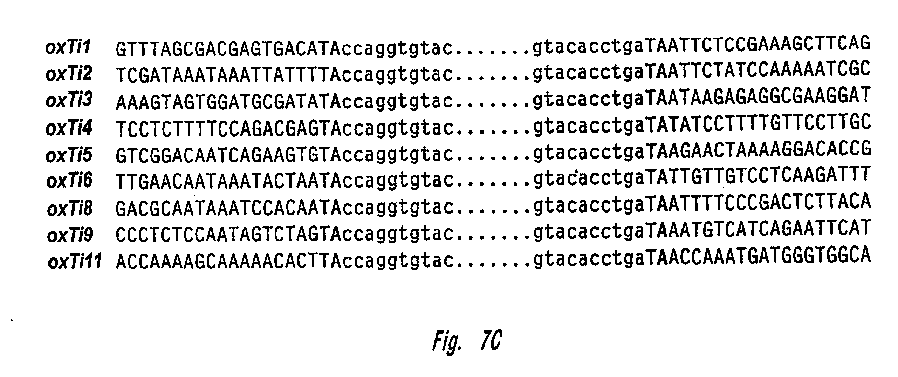

Figure 7 shows *Mos1* genomic insertions. (A) Southern blot probed with labeled *Mos1* DNA. Lanes 1 to 8, strains in which insertions were detected by PCR; insertions derived from an extrachromosomal array and *Mos* transposase expressed under the heat-shock promoter. *Mos1* presence was assessed by PCR using two primers located in the transposon oJL102 (SEQ ID NO: 1) and oJL103 (SEQ ID NO: 3). The absence of *D*. *mauritiana*-flanking sequence was checked using oJL102 (SEQ ID NO: 1) and oJL104 (SEQ ID NO: 2), while a PCR positive control was performed on each DNA sample using oligonucleotides located in the *cha-1* gene. The control lane is lin-15(n765) which had been used to build transgenic lines. Each lane contains 2 mg of Bgl II -digested genomic DNA. The *Mos1* probe (encompassing bases 1 to 174 of the transposon) was synthesized by PCR using the pBluescriptM13+/*Mos1* plasmid as a template. (B) Distribution of *Mos1* inserts on the physical map of the *C. elegans* genome. Black triangles: insertions from an extrachromosomal array. Open triangles: insertions from the integrated array oxIs25. Open circle: position of oxIs25, the integrated array of *Mos1* transposons. (C) DNA sequence of *Mos1* de novo insertions. Genomic fragments that flank the transposon left end were isolated by inverse PCR and sequenced. A primer was designed in the genomic region to the right of the insert and used with a *Mos1*-specific primer to amplify and sequence the right end flanking the fragment. At insertion sites TA dinucleotides (bold) were duplicated during the process of transposon integration. Lower case: *Mos1.* Upper case: genomic sequence. Ellipses: omitted sequence.

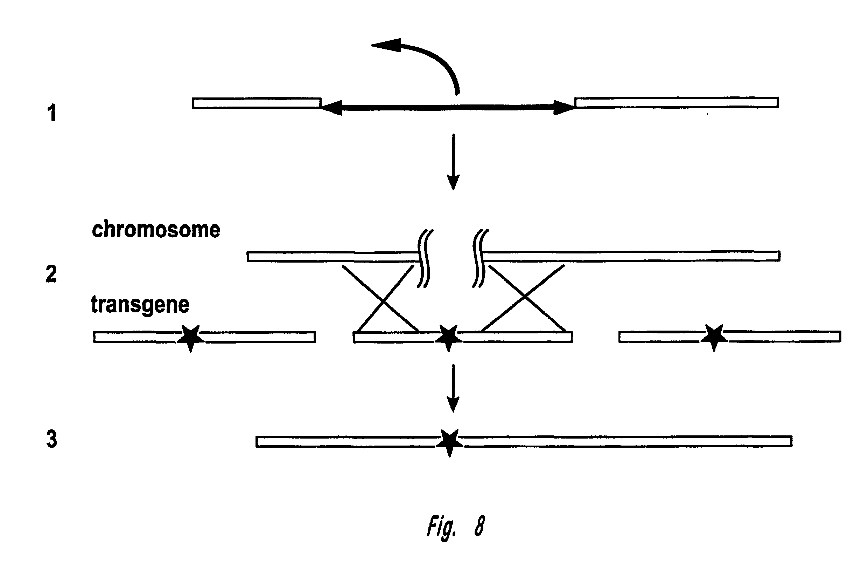

Figure 8 shows knock-in strategy where in *Mos1* excision causes a DNA double strand break, after which a transgene containing sequences homologous to the excision region pairs with the chromosome. Finally, the mutation contained in the transgene is copied into the chromosome.

These drawings only provide information concerning typical embodiments of the invention and are not, therefore, to be considered limiting of its scope.

### 5. DETAILED DESCRIPTION OF THE INVENTION

Disclosed herein are novel methods for generating and identifying mutations in *C. elegans,* and includes methods for introducing a transgene into the *C. elegans* germline. The present invention also includes methods for expressing transgenes in the *C. elegans* germline. The present invention also includes a transgene construct for expression in *C. elegans.* Also disclosed are methods for generating mutations by regulating the transposition of the endogenous or heterologous transposons in *C. elegans.* Also disclosed herein are methods for inserting single copy DNA into a *C. elegans* genome by introducing a transgene comprising FLP Recombination Target (FRT) sites into a *C. elegans* genome and causing recombination. FLP is a site-specific recombinase which efficiently catalyzes recombination between FRT sites that have been placed in the genome. K. G. Golic, S. L Linquist, Cell 44, 521 (1986). When FRT sites are in the same relative orientation within a chromosome, the FLP recombinase excises the intervening DNA from the chromosome. *See* Golic, Kent G., Generating Mosaics By Site-Specific Recombination, In Cellular Interactions In Development: A Practical Approach, 1-31 (D. A. Hartley, ed., Oxford Univ. Press 1993); and Plasterk R.H., Groenen J.T., Targeted Alterations of the Caenorhabditis elegans Genome by Transgene Instructed DNA Double Strand Break Repair Following Tcl Excision, EMBO J., 11:287-90 (1992). Other recombination systems, such as CRE/LOX, could also be used.

### DEFINITIONS

The term "heterologous" is used herein to include nucleic acid sequences such as coding sequences and control sequences that are not normally joined together, and/or are not normally associated with a particular cell. Thus, a heterologous region of a construct or vector is a segment of nucleic acid within or attached to another nucleic acid molecule that is not found in association with this other molecule in nature. For example, a heterologous region of a nucleic acid construct could include a coding sequence flanked by sequences not found in association with the coding sequence in nature (e.g., synthetic sequences having codons different from the native gene). Similarly, a cell transformed with a construct which is not normally present in the cell would be considered heterologous for purposes of this invention. The term includes, but is not limited to, a DNA sequence from another organism.

The term "transgene" is a heterologous sequence that is introduced into an organism. The term includes both sequences that integrate into one or more chromosomal locations of the organism and sequences that are maintained extrachromosomally, e.g., as episomes.

The term "regulable expression control element" includes promoters, polyadenylation signals, transcription termination sequences, upstream regulatory domains, origins of replication, internal ribosome entry sites, enhancers, and the like, which provide for the replication, transcription, and translation of a coding sequence in a recipient cell or in a cell of an organism. The term promoter refers to a DNA sequence that is capable of binding RNA polymerase and initiating transcription of a downstream (3' direction) sequence. Inducible promoters are promoters which are regulable. Such promoters may be regulated by, for example, temperature, small molecules, or developmental stages of an organism.

Inducible promoters include heat-shock promoters, which are induced by exposure to heat. Inducible promoters also include small molecule-regulated promoters. Other inducible promoters include promoters that are induced (or repressed) by tetracycline and its derivatives (Gossen & Bujard, Proc. Natl. Acad. Sci. USA 89:5547-5551 (1992)).

"Operably linked" refers to an arrangement of elements in which the components so described are configured so as to perform their usual function. Thus, control sequences such as regulable expression control elements operably linked to a coding sequence are capable of affecting the expression of the coding sequence. The control sequences need not be contiguous with the coding sequence so long as they function to direct the expression thereof. Thus, for example, intervening untranslated yet transcribed sequences can be present between a promoter sequence and the coding sequence and the promoter sequence can still be considered "operably linked" to the coding sequence.

For the purpose of describing the relative position of nucleotide sequences in a particular nucleic acid molecule throughout the instant application, such as when a particular nucleotide sequence is described as being situated "upstream," "downstream," "3'," or "5'," relative to another sequence, it is to be understood that it is the position of the sequences in the "sense" or "coding" strand of a DNA molecule that is being referred to as conventional in the art.

### GENERAL METHODS

In one embodiment, a method of regulated expression of a heterologous gene in cells of the germline of *C. elegans* comprises the steps of inserting a transgene construct into the *C. elegans,* wherein the construct comprises the heterologous gene operably linked to a promoter, and a 3' untranslated region of a gene that is expressed in the *C. elegans* germline and expressing the heterologous gene. Other embodiments further comprise the use of a promoter which is inducible, such as a heat-shock promoter or a tetracycline-regulated promoter. Yet other preferred embodiments comprise the removal of substantially all bacterial plasmid sequences and repeated sequences from the transgene. In certain other especially preferred embodiments, the method of the present invention comprises the addition of the 3' untranslated region (UTR) of the *glh-2* gene, which is expressed in the *C. elegans* gennline, to the 3' end of the transgene. A promoter, such as a *glh-2* promoter, which is a germline-specific promoter, may be used to drive expression of the transgene.

Also disclosed herein are transgene constructs for expression in *C. elegans* which comprise a heterologous gene operably linked to a promoter and a 3' untranslated region of a gene that is expressed in the *C. elegans* gennline. In certain embodiments, the promoter is active in the cells of the germline of *C. elegans.* In other embodiments, the promoter comprises an inducible promoter such as a heat-shock promoter or a tetracycline-regulated promoter expressed in the germline. Yet other embodiments comprise the removal of substantially all bacterial plasmid sequences and repeated sequences from the transgene. In certain preferred embodiments, the method of the present invention comprises the addition of a 3' untranslated region (UTR) of a *glh-2* gene, which is expressed in the *C. elegans* germline, to the 3' end of the transgene. A promoter, such as a *glh-2* promoter, may be used to drive expression of the transgene. In still other embodiments, the heterologous gene codes for a transposase. In certain preferred embodiments, the heterologous gene is a TC3A transposase gene.

Also disclosed are methods for generating mutations in the genome of *C. elegans* by using controlled mobilization of transposons. In certain embodiments, the method for generating mutations in the genome of *C. elegans* comprises the steps of introducing a transgene construct comprising a transposase gene which is operably linked to a regulable expression control element and a 3' untranslated region of a gene that is expressed in the *C. elegans* germline into *C. elegans* and expressing the transposase gene. Such methods allow the generation of mutants in which the mutated genes are tagged by the insertion of the transposon. PCR-based techniques permit fast identification of the transposon insertion that causes the mutation. Since the *C. elegans* genome has been entirely sequenced, sequencing of the genomic regions that flank the transposon allows immediate identification of the mutated gene.

In certain embodiments, transposons used in the method herein disclosed are endogenous transposons. Several different types of endogenous transposons are present in *C. elegans,* and these can be mobilized in mutator strains. *See, e.g.,* R. H. A. Plaster K, H. G. A. M. van Luenen, in C. elegans II, D. L. Riddle, T. Blumenthal, B. J. Meyer, J. R. Press, Eds. (Cold Spring Harbor Laboratory Press, New York, 1997) pp. 97-116. Mutator alleles have been useful in cloning *C. elegans* genes, particularly in early studies before the genome project reagents were widely available. In certain preferred embodiments, the endogenous transposons are Tc3 transposons. In yet other embodiments, the transposase gene is a TC3A transposase gene. In still other embodiments, the regulable expression control element is an inducible promoter, comprising in some embodiments a heat-shock promoter or a tetracycline-regulated promoter. Yet other embodiments comprise the removal of substantially all bacterial plasmid sequences and repeated sequences from the transgene construct. In certain preferred embodiments, the method of the present invention comprises the addition of the 3' untranslated region (UTR) of the *glh-2* gene, which is expressed in the *C. elegans* germline, to the 3' end of the transgene. In such embodiments, the regulable expression control element comprises a promoter, such as a *glh-2* promoter or a heat-shock promoter, which may be used to drive expression of the transgene.

In other embodiments, mutants may be generated by using controlled mobilization of heterologous transposons. Using a heterologous transposon allows researchers to tag mutated genes with a sequence that is unique in *C. elegans* genome. These tagged mutations will allow the rapid cloning of the mutated genes. The primary advantage over the endogenous transposon scheme is that this method avoids the isolation of irrelevant insertions of the endogenous *C. elegans* transposons. A further advantage is that the expression of the heterologous transposase would only mobilize the heterologous element, and thus mutations should only be due to insertions of these elements. Additionally, insertions could be stabilized by loss of the transposase-expressing construct.

In certain preferred embodiments, *Mos1,* a *mariner-like* transposon isolated from *Drosophila mauritiana,* is used. M. Medhora et al., Genetics 128:311-3 18 (1991). *See generally* D. L. Hartl et al., Annu. Rev. Genet. 31:337-358 (1997). *Mosl* is a member of the *mariner*/*Tcl* family and was initially identified in the fruitfly *Drosophila mauritiana.* J. W. Jacobson, M. M. Medhora, D. L. Hartl, Proc. Natl. Acad. Sci. USA 83, 8684 (1986). Like the other members of the *mariner*/*Tcl* family, *Mosl* contains a single open reading frame which encodes the transposase. The transposase binds to and cleaves at the inverted terminal repeats (ITRs) present at each end of the transposon. *See. e.g.* D. L. Hartl, A.R. Lohe, E. R. Lozovskaya, Annu. Rev. Genet. 31, 337 (1997); R. H. Plasterk, Z. Izsvak, Z. Ivics, Trends Genet. 15, 326-332 (1999). The *Mos1* transposase is the only protein necessary for transposition *in vitro.* L. R. Tosi, S. M. Beverly, Nucleic Acids Res. 28, 784 (2000). Because no additional factors are required for transposition, the *Mosl* transposon should be capable of transposition in heterologous species, and indeed the transposon has been mobilized in species evolutionarily distant from *Drosophila.* F. J. Gueiros-Filho, S. M. Beverly, Science 276, 1716 (1997); J. M. Fadool, D. L. Hartl, J. E. Dowling, Proc. Natl. Acad. Sci. USA 95, 5182 (1998); A Shennan, et al., Nat. Biotechnol. 16, 1050 (1998); C. J. Coates, N. Jasinskeine, L. Miyashiro, A. A. James, Proc. Natl. Acad. Sci. USA 95, 3748 (1998).

In other embodiments, the transposase gene comprises restriction sites 5' of the start codon, restriction sites 5' of the stop codon, and an artificial intron in the transposase gene open reading frame. Other preferred embodiments involve a regulable expression control element which comprises an inducible promoter such as a heat-shock promoter or a tetracycline-regulated promoter. Yet other preferred embodiments comprise the removal of substantially all bacterial plasmid DNA sequences and repeated sequences from the transgene construct. In certain preferred embodiments, the method of the present invention comprises the addition of the 3' untranslated region (UTR) of the *glh-2* gene, which is expressed in the *C. elegans* germline, to the 3' end of the transgene. In such embodiments, the regulable expression control element may comprise a promoter, such as a *glh-2* promoter, a *myo-3* promoter or a heat-shock promoter, which may be used to drive expression of the transgene.

In yet other embodiments, the method disclosed herein includes engineering the transposon to carry a heterologous DNA sequence into a *C. elegans* chromosome. Certain embodiments of the method herein disclosed may comprise the steps of introducing a transposon into the *C. elegans,* wherein the transposon comprises the heterologous DNA sequence; introducing a transgene construct into the *C. elegans,* wherein the construct comprises a transposase gene which is operably linked to a promoter and a 3' untranslated region of a gene that is expressed in the *C. elegans* germline; and expressing the transposase, such that the transposon integrates as a single copy into a *C. elegans* chromosome.

In some embodiments, the transposon may be modified to contain bacterial plasmid DNA sequences. Such sequences may simplify cloning of mutated genes into bacteria from *C. elegans* genomic DNA preparations. In yet other embodiments, the transposon may carry a gene useful for selection or screening purposes.

In certain preferred embodiments, FRT/FLP or CRE/LOX recombination sites could be inserted into the transposon. One of skill in the art would appreciate that an engineered transposon carrying such recombination sites would facilitate insertion of single copy DNA into the *C. elegans* genome. In other embodiments, the transposon could include polyadenylation sites or transcriptional terminators.

In yet other preferred embodiments, the promoter is inducible. In such embodiments, inducible promoters such as a heat-shock promoter, may be used. Yet other preferred embodiments comprise the removal of substantially all bacterial plasmid DNA sequences and repeated sequences from the transgene construct. In certain preferred embodiments, the method of the present invention comprises the addition of the 3' untranslated region (UTR) of the *glh-2* gene, which is expressed in the *C. elegans* germline, to the 3' end of the transgene. In such embodiments, the regulable expression control element may comprise a promoter, such as a *glh-2* promoter, a *myo-3* promoter or a heat-shock promoter, which may be used to drive expression of the transgene.

### 6. EXAMPLES

The following examples are given to illustrate several embodiments which have been made within the scope of the present invention. It is to be understood that these examples are neither comprehensive nor exhaustive of the many types of embodiments which can be prepared in accordance with the present invention.

### Example I - Mobilization of Endogenous Tc3 Transposons

About 15 copies of the Tc3 transposon are present in the genome of the wild-type *C. elegans* N2 strain. These transposable elements are inactive in wild-type animals. Our goal is to cause specific mobilization of the endogenous Tc3 copies by expressing the TC3A transposase in the germline. New Tc3 insertions will be used as tags to clone the genes which they have disrupted.

### I - TC3A expressed under the ced-9 promoter causes somatic and germline hops:

The TC3A transposase gene has been cloned behind a *ced-9* promoter. This construct has been coinjected with linearized *C. elegans* genomic DNA and the *lin-15(+)* plasmid into a *lin-15(-)* strain and unstable transgenic strains have been obtained. Transposase activity was assayed by testing whether the construct could excise a Tc3 element from the *unc-22* gene and restore the function of the locus. The *ced-9::Tc3A* arrays have been crossed into *unc-22(r750::Tc3)*; *lin-15(n765ts)* background. Wild-type revertants have been recovered from Unc-22 F1 animals, suggesting functional expression of the TC3A transposase. One of these extrachromosomal arrays was integrated into a chromosomal location to generate the insertion *oxls17[ced-9::Tc3A; lin-15(+)]*.

*oxls17* was mapped on the X chromosome and functionally characterized. *lin-15(n765ts);oxIsl7[ced-9::Tc3A; lin-15(+)]* males were crossed to *unc-22(r750:: Tc3); lin-15(n765ts)* hermaphrodites. Heterozygous nonUnc nonLin hermaphrodites were cloned and allowed to self-fertilize. It was expected that among the progeny of these animals, there would be found 1/4 Unc animals homozygous for *unc-22(r750::Tc3).* Of those Unc individuals, 3/4 should be either homozygous or heterozygous for *oxIs17 i.e.* nonLin. It was observed, however, that the Unc nonLin animals were greatly under-represented; instead, there were many more nonUnc individuals. Since the *ced-9* gene is ubiquitously expressed, it was reasoned that TC3A could be present not only in the germline but also in somatic cells and could cause somatic reversion of the Unc phenotype. To test this hypothesis, nonUnc nonLin individuals were cloned, assuming that a fraction of them could be homozygous for *unc-22(r750::Tc3)* despite their wild-type phenotype. Self-progeny of these animals were scored. Individuals heterozygous for *oxIs17* segregated 1/4 Lin animals (which no longer expressed the TC3A transposase). In this category, plates were identified in which 100% of the Lin worms were Unc while almost 100% of the nonLin were nonUnc. Hence, the parent hermaphrodite must have been homozygous *unc-22(r750::Tc3)* mutant although its phenotype was wild-type. These data demonstrate that *ced-9::Tc3A* causes somatic reversion of the *unc-22(r750::Tc3)* locus at high frequency.

Rare nonUnc Lin were looked for among the Lin animals generated by self-fertilization of *unc-22(r750::Tc3); lin-15(-); oxIs17*/*+* hennaphrodites to detennine germline reversion rates. Since the Lin worms had lost *oxIs17* and had no TC3A transposase expressed somatically during development, the only way to revert the Unc phenotype was to receive one reverted copy of the *unc-22* locus. This reversion event had to occur during germline development. Rare nonUnc Lin progeny were identified among Unc Lin progeny (experiment #1: nonUnc Lin in 61 Lin total; exp. #2: 2/106; exp. #3: 4/203). It was concluded that *ced-9::Tc3A* causes an approximately 2% reversion rate of the *unc-22(r750::Tc3)* locus in the germline.

### 2 - Expression of TC3A using a glh-2 promoter:

Since the somatic reversion caused by *ced-9::Tc3A* causes a discrepancy between the phenotype and the genotype of an individual carrying a locus disrupted by a Tc3 insertion, a TC3A expression vector was designed based on the germline-specific *glh-2* gene (gift of Karen Bennett). A plasmid containing a glh-2 genomic fragment is able to rescue the Glh-2 mutant phenotype and is therefore likely to be expressed in the germline. The *glh-2* open reading frame was deleted and replaced by a multiple cloning site to generate an expression cassette that retains *glh-2* promoter and 3' untranslated regions. *Tc3A* was inserted to generate *glh-2::Tc3A.* This construct has been coinjected with linearized *C. elegans* genomic DNA and the *lin-15(+)* plasmid into a *lin-15(-)* strain and several unstable transgenic strains have been obtained. A plasmid driving strong expression of the Green Fluorescent Protein (hereinafter "GFP") in the coelomocytes (gift of Piali Sengupta) has also been incorporated in this array. This allows monitoring of the presence of the array in a *lin-15(+)* background based on GFP expression.

As described above, these arrays have been crossed into *unc-22(r750::Tc3)*; *lin-15(n765ts)* background. In contrast to the *ced-9::Tc3A* experiments, no somatic reversion events were observed. Germline reversion events were observed in the progeny of *unc-22*; *oxEx[glh-2:: Tc3A]* hennaphrodites (experiment #1: 2/1914 total scored animals; experiment #2: 5/4312). It was concluded that *glh-2::Tc3A* causes a 0.1% reversion rate of the *unc-22(r750::Tc3)* locus in the gennline.

### Example 2 - Expression of the Mariner Transposase in C. elegans

A mutagenesis strategy was also developed that uses the mariner transposon from the hornfly (gift of David Lampe and Hugh Robertson). Mariner transposons from *Drosophila* are related to *C. elegans* Tc transposons. In fact, members of the Tc/*mariner* family of transposable elements have been identified in a broad range of species. R. H. A. Plasterk & H. G. A. M. van Luenen, Transposons, in C. elegans II 97-116 (D.L. Riddle et al. eds., 1997). Horizontal transfer may be responsible for the broad distribution of this family of transposable elements. Horizontal transfer implies that specific host factors are not required for transposition and biochemical characterization has borne this supposition out. Purified transposase is able to catalyze the transposition of mariner or of Tc elements from a host plasmid to a target plasmid. D. J. Lampe et al., EMBO J. 15:5470-5479 (1996); J. C. Vos et al., Genes Dev. 10:755-761 (1996). This has enabled researchers to mobilize mariner elements from *Drosophila* in other Dipteran species. T. G. Loukeris et al., Proc. Natl. Acad. Sci. USA 92:9485-9489 (1995); T. G. Loukeris et al., Science 270:2002-2005 (1995); A. R. Lohe & D. L. Hartl, Genetics 143:365-374 (1996). Recently, a mariner element from *Drosophila* has been mobilized in *Leishmania,* which represents a trans-kingdom transposition. F. J. Gueiros-Filho & S. M. Beverly, Science 276:1716-1719 (1997). Thus, it was possible that mariner would be active in *C. elegans* as well.

A plasmid encoding the mariner transposase HIMAR1 was received from David Lampe and Hugh Robertson. First, the transposase coding sequence was engineered to allow for efficient expression in *C. elegans.* Restriction sites were inserted immediately upstream to the start codon and just before the stop codon to facilitate subcloning of the fragment in various expression vectors. An artificial intron was inserted in the open reading frame since the presence of introns improves the expression level of transgenes in *C. elegans.*

Engineered *Himar1* was placed under the control of the muscle-specific promoter *myo-3.* The *myo-3::Himar1* construct was injected with the *lin-15(+)* plasmid into a *lin-15(-)* strain and unstable transgenic strains obtained. Expression of the HIMAR1 transposase was examined first by Western Blot. Extracts were prepared from *oxEx[myo-3::Himar1*; *lin-15(+)]* worms, run on a denaturing acrylamide gel and transferred to a nitrocellulose membrane. The membrane was probed with previously characterized antibodies that recognize the HIMAR1 protein (provided by David Lampe and Hugh Robertson). In extracts of transgenic wonns, an approximately 42 kD protein which corresponds to the expected molecular weight of HIMAR1 was detected. The signal was absent from non-transgenic worm extracts. Using the same antibodies, the protein was visualized in situ using immunofluorescence on *oxEx[myo-3::Himar1*; *lin-15(+)]* wonns. Intense immunoreactivity was detected which was restricted to the nuclei of muscle cells. These data indicate that the HIMAR1 mariner transposase is expressed and properly targeted to nuclei in *C. elegans* cells.

### Example 3 - Germline Expression of the Transposase Using the glh-2 Promoter

Generation of heritable *Mos1* insertions would require expression of the *Mos* transposase in the germline. However, expression of transgenes in the germline of *C. elegans* is not possible using standard techniques. Typically, transgenic worms are generated by injecting plasmid DNA into the gonads of *C. elegans* (C. C. Mello, J. M. Kramer, D. Stinchcomb, V. Ambros, Embo J. 10, 3959 (1991)). These fragments then form a simple array of repeated DNA segments. Although gene expression is robust in somatic tissues, such simple arrays are not expressed in the germline or are silenced after a few generations. Co-injection of genomic DNA with plasmid DNA improves germline expression, presumably by preventing tandem repeats in the array. W. G. Kelly, S. Xu, M. K. Montgomery, A. Fire, Genetics 146, 227 (1997). To express the *Mos* transposase in the germline, an expression vector containing the promoter and the 3' UTR of the *glh*-2 gene was built. This gene encodes a germline helicase which is specifically expressed in the gonad. M. E. Gruidl, et al., Proc. Natl. Acad. Sci. USA 93, 13837 (1996). Transgenic lines carrying extrachromosomal arrays of the *glh-2::Mos transposase* construct were generated by microinjection. To maximize expression in the germline, constructs were isolated from plasmid vector sequences and were coinjected with fragmented genomic DNA. (The *Mos* transposase coding sequence was introduced between the promoter and the 3' UTR of *glh-2.* Specifically, this construct (pJL9) contains 2.2 kb of the *glh-2* genomic sequence immediately upstream of the translation start site (nt 29,882 to 32,095 in cosmid C55B7), an Mlu I-Nhe I cloning site, and 0.8 kb of sequence immediately downstream of the *glh-2* stop codon. An Mlu I-Nhe I fragment containing the *Mos* transposase was subcloned into pJL9 to generate the *glh-2::MosTransposase* construct. *lin-15(n765)* hermaphrodites were injected with a Spe I - Kpn I fragment of *glh-2::MosTransposase* (injection concentration 10 ng/µl), with *lin-15(*+*)* (EKL15) and *ofm-1::gfp* (pPD97/98) fragments and N2 wonn genomic DNA as described above for the generation of the *oxEx166[hsp::MosTransposase]* array.

Transposase expression in the germline was determined by assaying for excision of transposons from a defined chromosomal location. Specifically, the *Mos1*-containing extrachromosomal array was integrated into chromosome V to generate *oxIs25[Mos1;rol-6(sd)].* The oxIs25 array was mapped less than 0.54 map units from dpy-11. Heterozygous oxIs12/dpy-11 worms were generated. These animals largely segregated Dpy and Rol progeny as expected for these closely linked markers (Figure 6). However, when the *glh-2::Mos* Transposase transgene was crossed in, approximately 16% of the nonDpy progeny were nonRol (15.7% ± 0.9, mean ± SEM, n=44 plates). The nonRol phenotype was stably inherited. It was hypothesized that in those worms, the *Mos* transposase excised *Mos1* from the integrated array. The resulting DNA breaks were responsible for catastrophic excision of the entire locus, including interspersed rol-6 copies. The correlated loss of rol-6(sd) and *Mos1* was confirmed by PCR, in which NonRol individuals were cloned, selfed and DNA was purified from the progeny. *Mos1*-containing fragments were detected by PCR using one primer complementary to *Mos1* (oJL102: 5'-CAACCTTGACTGTCGAACCACCATAG (SEQ ID NO: 1)) and one primer complementary to *D. mauritiana-flanking* DNA (oJL104: 5'-ACAAAGAGCGAACGCAGACGAGT (SEQ ID NO: 2)). Of 188 nonRol wonns, only one individual retained a copy of the *Mos1* fragment that was initially present in the transgene. Based on the phenotypic reversion of the Rol phenotype, it was calculated that I in 5 chromosomes experienced catastrophic excision of the transgene (20.9% ± 1.1 %, mean ± SEM, n=44 plates). The probability p of a single chromosome containing the array of *Mos1* elements transgene experiencing "catastrophic excision" can be derived from a Punnett square where the ratio R of nonRol worms over the total number of the progeny: R = 1/4p + 1/4p + (1/2p)². These results demonstrated that the glh-2-based expression vector expressed the transposase in the germline and that the *Mos1* transposon in the chromosome was recognized as a substrate.

To determine if excision of *Mos1* from the array was associated with insertion in the genome, the progeny of animals expressing the transposase in the germline were screened for de novo insertions. Specifically, using PCR, the presence of the *Mos1* element in the absence of the *Drosophila* sequences which flank the transposon in the array was assayed. *Mos1* presence was assessed by PCR using two primers located in the transposon (oJL102 (SEQ ID NO: 1) and oJL103: 5'-TCTGCGAGTTGTTTTTGCGTTTGAG (SEQ ID NO: 3)). The absence of *D. mauritiana*-flanking sequence was checked using oJL102 (SEQ ID NO: 1) and oJL104 (SEQ ID NO: 2) as described above. In addition, a PCR positive control was performed on each DNA sample using oligonucleotides located in the *cha-1* gene. Because the integrated array containing unmobilized transposons also contained rol-6(sd), insertions were sought in nonRol progeny; specifically, either nonRol animals that experienced catastrophic excision of the array or Dpy progeny (Figure 6B) were analyzed. Insertions were identified in 1% of nonRol progeny (2/227) and in 10% of Dpy progeny (11/116 F1+F2 Dpys). These results demonstrated that transposition of *Mos1* could be achieved in the *C. elegans* germline. However, it was observed that high rates of excision were not accompanied by high rates of insertion; these results support previous data indicating that these two processes are not coupled. H. G. van Luenen, S. D. Colloms, R. H. Plasterk, Cell 79, 293 (1994).

Using integrated arrays as a source of transposons prevents the easy recovery of new insertions that occur on the same chromosome; this bias could be circumvented by using an extrachromosomal array of transposons. In addition, extrachromosomal arrays are not completely stable in meiosis, which makes the isolation of strains lacking unmobilized transposons easy after mobilization. Therefore, it was tested whether *Mos1* could be mobilized from an extrachromosomal array into the chromosomes. Specifically, the *glh-2::Mos* Transposase construct was used to mobilize transposons from a *Mos 1*-bearing array (*oxExI64[Mos1*; *rol-6(sd)]*). The nonRol progeny from double transgenic animals (*oxEx167[glh-2::MosTransposase]*; *oxEx164(Mos1; rol-6(sd)]*) were analyzed for transposition events using PCR. An insertion frequency of 1% (3 insertions/ 302 progeny, Table 1) was detected. Thus, these results closely match those obtained for integrated arrays.

Table 1. Frequencies of *Mos1* genomic insertions from an extrachromosomal array. nonRol progeny of *oxEx164[Mos1*; *rol-6(sd)]*; *oxEx[MosTransposase]* were analyzed by PCR for the presence of *Mos1* and the loss of the *Drosophila*-flanking sequences present in the donor plasmid. *Mos1* presence was assessed by PCR using two primers located in the transposon (oJL102 (SEQ ID NO: 1) and oJL103: 5'-TCTGCGAGTTGTTTTTGCGTTTGAG (SEQ ID NO: 3)). The absence *of D. mauritiana*-flanking sequence was checked using oJL102 (SEQ ID NO: 1) and oJL104 (SEQ ID NO: 2) as described above. In addition, a PCR positive control was performed on each DNA sample using oligonucleotides located in the *cha-1* gene. When heat-shocked (1 hour at 35°C) P0s were moved to fresh plates and eggs were collected for the next 24 hours. *During experiment #5, the stability of the *oxEx1G4[Mos1*; *rol-6(sd)]* transgene reached 75%, while in previous experiments, it was approximately 20%.

| Transposase construct | Transposition frequency | | | |
|---|---|---|---|---|
| | no heat-shock | | with heat-shock | |
| *glh-2::MosTransposase* | exp #1: 2/108 | 1.9% | exp #1: ND | |
| | exp #2: 0/104 | 0% | exp #2: ND | |
| | exp #3: 1/90 | 1.1% | exp #3: 0/65 | 0% |
| *hsp::MosTransposase* | exp #1: ND | | exp #1: 4/65 | 6.2% |
| | exp #2: 0/33 | | exp #2: 3/98 | 3.1% |
| | exp #3: 0/39 | | exp #3: 6/87 | 6.8% |
| | exp #4: ND | | exp #4: 5/85 | 5.9% |
| | exp #5: 0/44* | | exp #5: 15/34* | 44.1% |

### Example 4 - Mos1 - Mobilization in the Germline Using a Heat-Shock Promoter

The *glh-2* promoter expresses the transposase in the germline constitutively. Constitutive expression of the transposase has two disadvantages. First, crosses must be set up fresh every generation to guarantee that the array remains intact and does not accumulate inherited changes. Second, because the tranposase was expressed in the germline early in development, events identified in the progeny might not be independent but might have occurred when the germline was still comprised of only few cells. Expression limited to adults can be achieved by using a heat-shock promoter. Expression of the transposase could be induced after a strain containing the transposase and transposons had been propagated and expanded to many animals. In addition, heat-shocking animals with mature germlines would maximize the independence of insertion events. Animals expressing the transposase under the control of the heat-shock promoter and bearing the integrated transposon (*oxIs25*/*dpy-11*; *oxEx166[hsp::Mos Transposase]*) were heat-shocked. P0s could only be heat-shocked for 45 minutes; such animals were almost paralyzed, stopped eating and had low brood sizes. Longer heat-shock caused the animals to die. It was speculated that this lethality is due to high rates of transposition in somatic cells. Ubiquitous expression of the transposase would cause double strand breaks in the chromosome at the site of integration in every cell which may cause cell cycle arrest or apoptosis. G. Evan, T. Littlewood, Science 281, 1317 (1998).

F1 progeny were analyzed for catastrophic excision, that is, for the appearance of nonRol nonDpy progeny (Figure 6A). In contrast with results obtained using the *glh-2* expression vector, catastrophic excision was not observed. Only rare nonRol progeny were generated which were likely to be the result of recombination between the array and the dpy marker (Figure 6B). However, by analyzing the Dpy progeny of heat-shocked animals, it was discovered that the heat-shock construct caused the efficient insertion of *Mos* into new locations in the genome. Approximately 27% (25/94) of F1 or F2 Dpy worms carried novel transposon insertions (Figure 6B). In addition to novel insertions, recombinant chromosomes containing both the dpy marker and the original array arose at high frequency. These were identified as Dpy animals containing transposons flanked by *Drosophila* DNA. This hotspot for recombination is likely to arise as a result of double strand breaks introduced into the array by the transposase. A. R. Lohe, C. Timmons, 1. Beerman, E. R. Lozovskaya, D. L. Hartl, Genetics 154, 647 (2000).

It was then tested whether transposition could occur from an extrachromosomal array using the heat-shock promoter construct to express the transposase (oxEx166; *oxEx164[Mosl; rol-6(sd)]*)*.* Hermaphrodites bearing both arrays were heat-shocked as young adults. The nonRol progeny were analyzed by PCR for transposition events. New insertions were observed in 8.9% of the F1 (33/369 progeny, Table 1). Since transposition could have occurred into the transposase-containing array, F2 animals that lost this array were isolated from eight transposon-bearing strains. Genomic DNA was prepared from these strains and analyzed for the presence of *Mos1.* The transposon was still detected in all eight strains, thus demonstrating that the transposon had not inserted in the array. No insertions could be detected in 116 F1 clones derived from non-heat-shocked parents. The frequency of transposition was low but one of the main limiting factors is the stability of the extrachromosomal array that is used as a transposon source. The initial experiments were performed when the array was only 20% stable and transposition frequencies were in the range of 5%; when the array matured and was about 75% stable, transposition frequency reached 44%. Generating more stable extrachromosomal arrays could increase the frequencies of transposition.

The heat-shock promoter was able to drive expression *of Mos* transposase in the germline and to promote transposition events at a higher rate than obtained using the *glh-2* construct. Temperature has been shown to affect transposition frequency in other organisms. D. Garza, M. Medhora, A. Koga, D. L. Hartl, Genetics 128, 303 (1991). One possible explanation for the efficient transposition observed after heat-shock is that chromatin structure is somehow altered by the heat-shock. Therefore, it was tested whether heat-shock itself could account for the difference in transposition frequencies. Parents with extrachromosomal arrays carrying the g/h-2-transposase construct and carrying the transposon were heat-shocked and progeny were tested for transposition. The frequency of transposition was not improved by the heat-shock treatment (Table 1). Thus, heat-shock itself does not facilitate efficient transposition.

### Example 5 - Transposition in Somatic Cells

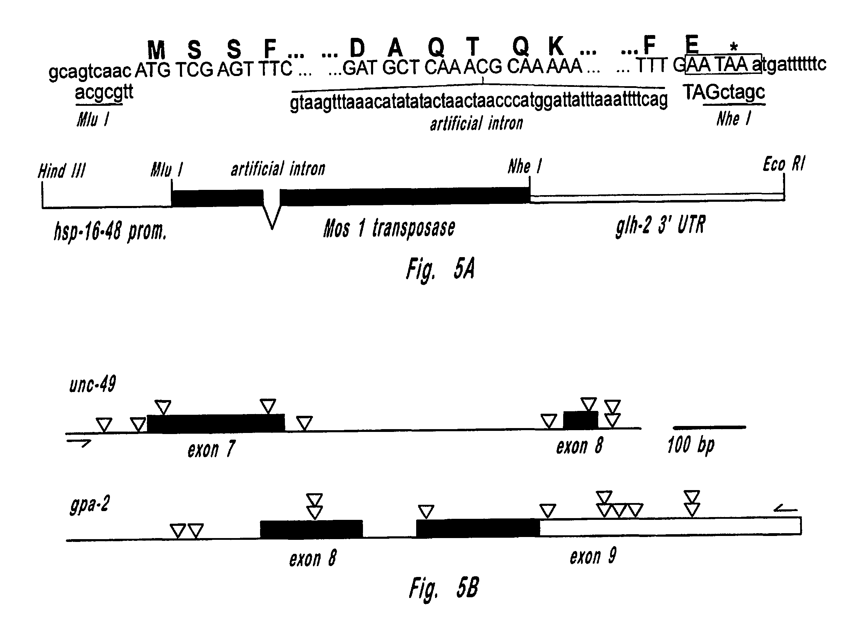

To determine whether the *Mos1* element could be mobilized in *C*. *elegans* cells, *Mos1* transposition in somatic cells was first analyzed. The gene encoding the *Mos1* transposase was engineered to improve expression in the worm and placed under the control of a heat-shock promoter. The *Mos* transposase encoding sequence was PCR amplified out of pBluescribe M13+/*Mos1* (M. Medhora, K. Maruyama, D. L. Hartl, Genetics 128, 311 (1991), modified as described in Figure 5A and subcloned as a Mlu I-Nhe I fragment between the *hsp-16-48* promoter (H. G. van Luenen, S. D. Colloms, R. H. Plasterk, Embo J. 12, 2513 (1993)); E. P. Candido, et al., Genome 31, 690 (1989) and the *glh-2* 3' untranslated region (fragment 35383 to 36190 in cosmid C55B7) (M. E. Gruidl, et al., Proc. Natl. Acad. Sci. U S A 93, 13837 (1996) (Figure 5A). The resulting construct was used to generate the extrachromosomal array *oxEx166[hsp::MosTransposase].* (lin-15(n765) hermaphrodites were injected in the syncitial gonad with a mixture of the following gel-purified fragments: a Hind III-Eco RI fragment of *hsp::Mos Transposase* (injection concentration: 10 ng/µl), a Pst I-Bsi WI fragment of the *ofm-I::gfp* construct (pPD97/98) that expresses GFP in the coelomocytes (gift of Piali Sengupta) (injection concentration: 5 ng/µl) and a Kpn I-Eag I fragment of EKL15(*lin-15* +) (S. G. Clark, X. Lu, H. R. Horvitz, Genetics 137, 987 (1994)) (injection concentration: 10 ng/µl). Plasmid backbones were removed from all purified fragments. Eco RV-digested N2 worm genomic DNA was coinjected at a concentration of 70 ng/µl. Another extrachromosomal array, *oxEx164[Mos1; rol-6(sd)],* contained the *Mos1* transposon. *(lin-15(n765)* hermaphrodites were injected with a 2.2 kb Xho I-Hind III fragment of pBluescribe *M13+*/*Mos1* that contains the 1.3 kb *Mos1* element flanked by *D. simulans* sequences (M. Medhora, K. Maruyama, D. L. Hartl, Genetics 128, 311 (1991) (injection concentration: 10 ng/µl) and a 2.2 kb *rol-6(sd)* fragment of pRF4 (J. M. Kramer, R. P. French, E. C. Park, J. J. Johnson, Mol. Cell. Biol. 10, 2081 (1990) (injection concentration: 10 ng/µl). Eco RV-digested N2 worm genomic DNA was coinjected at a concentration of 80 ng/µl to increase the complexity of the array; this array also contained the dominant genetic marker *rol-6(sd)* which causes animals to roll instead of swimming in a sinusoidal fashion. These two strains were crossed and progeny carrying both arrays were heat-shocked as young adults. After 12 hours, the heat-shocked animals were harvested and genomic DNA was prepared. *Mos1* transposition was detected using the strategy developed by van Luenen et al. *See* H. G. van Luenen, S. D. Colloms, R. H. Plasterk, Embo J. 12, 2513 (1993). Specifically, insertions were identified by PCR amplification using one set of primers complementary to the transposon and another set complementary to an arbitrary target gene. DNA purification and PCR were performed as described in H. G. van Luenen, S. D. Colloms, R. H. Plasterk, Embo J. 12, 2513 (1993). The primers in *Mos1* were oJL88 (5'-CGCATGCGGCTTACTCAC (SEQ ID NO: 4)) first PCR; and oJL89 (5'-GGCCCCATCCGATTACCACCTA (SEQ ID NO: 5)) second PCR. Primers in *unc-49* were oJL19 (5'-GCGAAACGCATACCAACTGTA (SEQ ID NO: 6)) first PCR; and oJL20 (5'-TTCATGCCGAAAAGCAGGCGT (SEQ ID NO: 7)) second PCR. Primers in *gpa-2* were the same as described in H. G. van Luenen, S. D. Colloms, R. H. Plasterk, Embo J. 12, 2513 (1993). PCR products were gel-purified and sequenced using oJL89 (SEQ ID NO: 5) as a primer. A PCR product can be obtained only if a transposon has integrated into the target gene. The method is sensitive enough to detect a single insertion in the target gene in a single somatic cell of an adult animal. Insertions in two genes were assayed: the *gpa-2* gene which encodes a G protein subunit (R. R. Zwaal, J. E. Mendel, P. W. Sternberg, R. H. Plasterk, Genetics 145, 715 (1997)), and the *unc-49* gene which encodes a GABA receptor. B. A. Bamber, A. A. Beg, R. E. Twyman, E. M. Jorgensen, J. Neurosci. 19, 5348 (1999). *Mos1* insertions were detected in both genes (2.5 ± 1.0 inserts in 10 ng of genomic DNA, mean ± S.D., n=5 experiments). Given that the maximal distance of the inserts from our gene primers was approximately I kb, it was estimated that an average of 10 insertions occurred per cell in heat-shocked animals. Insertions were also detected at low frequency in worms that contained the transposon array but lacked the transposase expression construct (0.09 insertions in 10 ng DNA, n=2 experiments). These data indicated that low levels of *Mos1* transposase were expressed from the intact *Mos1* transposons in the extrachromosomal array.

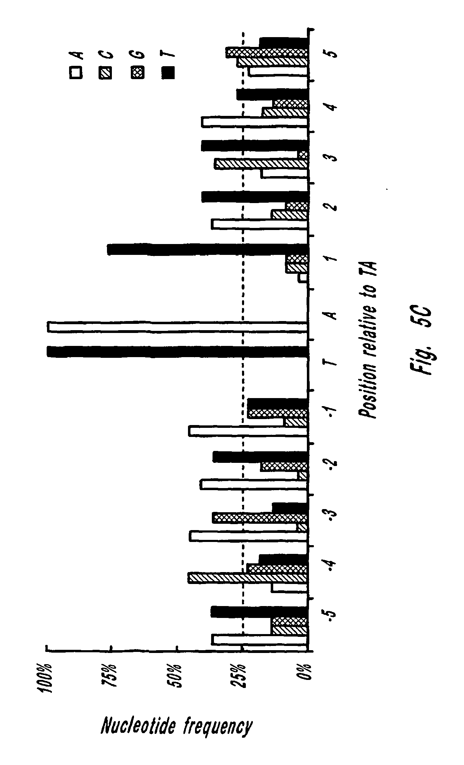

To demonstrate that these transposon insertions represented *bona fide* transposition events, PCR products were gel-purified and the sequence of the insertion sites from the somatic transposition assays was determined. In all cases, the *Mos1* inverted terminal repeats were complete, the *Drosophila* sequences that flanked *Mos1* in the donor plasmid were no longer present, and the insertions all took place at a TA dinucleotide. Transposon insertions were distributed uniformly in exons, introns and 3' noncoding sequences of *gpa-2* and *unc-49* (Figure 5B). Comparison of 22 insertion sites did not reveal a strong consensus site apart from a bias toward a T at position +1 with respect to the TA dinucleotide (Figure 5C). These data demonstrated that *Mos1* can hop into *C*. *elegans* chromosomes and that the transposase was sufficient to catalyze insertion without *Drosophila* host factors.

### Example 6 - Introduction of Mariner Transposon Copies into the C. elegans Genome

The full-length copy of the hornfly mariner transposon *Autmar* was gel-purified to remove non-nematode plasmid sequences. Purified *Autmar* was injected with linearized *C. elegans* genomic DNA and the *rol-6(dm)* plasmid into *lin-15(n765ts)* worms and unstable transgenic strains were recovered. Due to the presence of *rol-6(dm)* in the array, transgenic animals roll instead of displaying normal sinusoidal locomotory movements. These animals are Lin when grown at the nonpermissive temperature because they are genotypically *lin-15(-).* This array was integrated into a chromosomal location to generate the *oxIs21* insertion. *oxIs21* was mapped to chromosome X, 2.5 m.u. away from the *lon-2* locus.

### Example 7 -The Mariner Transposase Can Excise Mariner Transposons from C. Elelegans Chromosomes in the Germline

Engineered *Himar1* was inserted in the *glh-2* germline expression cassette described above. The *glh-2::Himar1* construct was co-injected with linearized *C*. *elegans* genomic DNA and the *lin-15(+)* plasmid into *lin-15(n765ts)* worms. The *oxEx115* extrachromosomal array is transmitted at each generation to a large fraction of the progeny.

*lin-15(n765ts); oxEx119[glh-2::Himar1; lin-15(+)]* males were crossed into *lin-15(n765ts) oxIs21[Autmar; rol-6(sd)]* hermaphrodites. As predicted, animals of the cross-progeny were Rol nonLin. At the next generation, it was expected that 1/3 of the Rol animals would be found to be homozygous for *oxIs21.* However, among 48 Rol nonLin cloned individuals, none segregated more than approximately 75% Rols, while 6 of 15 Rol Lin hermaphrodites segregated 100% Rol progeny. After careful characterization of the progeny of parent animals exhibiting various phenotypes, it was concluded that *oxEx115* could elicit the reversion of the Rol phenotype. Presumably, the reversion is caused by excision of the *Autmar* transposons from the integrated array which in turn leads to loss of the adjacent *rol-6(dm)* genes by imprecise repair of the locus. It was concluded that the mariner transposase can excise mariner transposons from *C*. *elegans* chromosomes in the germline.

### Example 8 - Mobilization of a Heterologous Mariner Transposon in the C. elegans Genome Materials and Methods:

### Reagents

*Mos1*-containing strain:

The transgenic strain EG1638 that contains *Mos1* has been generated by coinjection of *lin-15(n765)* worms with:
- the 2.2 kB Xho 1-Hind III fragment of pBluescribe M 13+/*Mos1,* M. Medhora et al., Genetics 128:311-318 (1991); (injection concentration: 10 ng/µl)
- the 2.2 kB Hind III *rol-6* rescuing fragment containing the semi-dominant mutation *rol-6(su1006),* J. M. Kramer et al., Mol. Cell. Biol. 10:2081-2089 (1990); (injection concentration: 10 ng/µl)
- EcoRV-digested genomic DNA prepared from N2 worms (injection concentration: 80 ng/µl)

The resulting strain *lin-15(ts); oxEx164[Mos1; rol-6(sd)]* exhibits a Rol Muv phenotype when grown above 20°C. The Muv phenotype is not expressed when worms are grown at 15°C.

*Mos1* transpose-expressing strain:

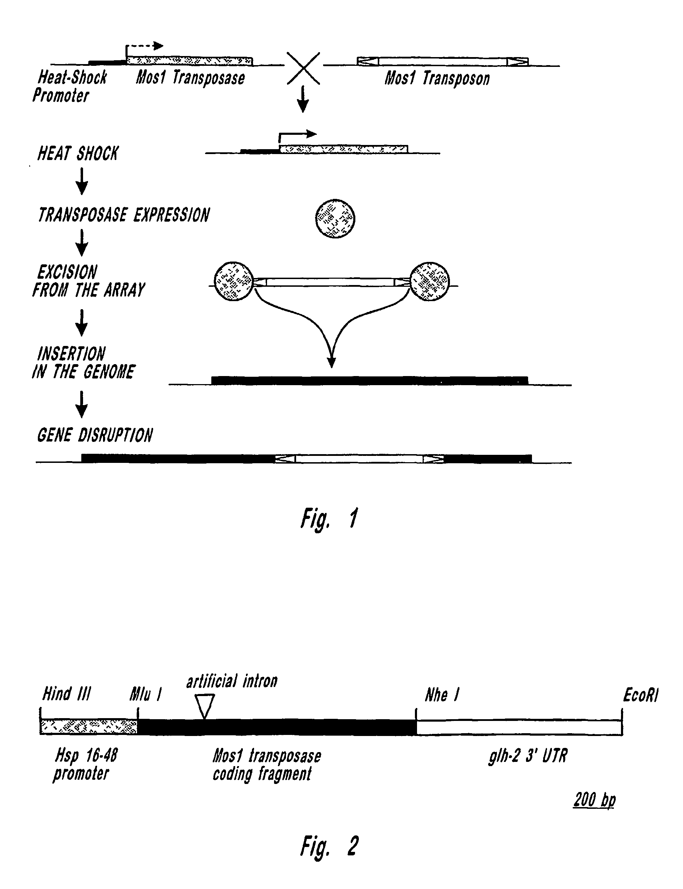

As shown in Figure 2, the expression vector pJL44 (*HSP::MosTase::glh-2*) contains the following elements:
- a 377 bp Hsp16-48 heat-shock promoter fragment recovered by PCR from pRP176, H. G. van Luenen et al., EMBO J. 12:25 3-2520 (1993), using the oligos oJL21 5'-CGAAGCTTGCTGGACGGAAATAGTGG (SEQ ID NO: 19) and oJL22 5'-CGACGCGTTCTTGAAGTTTAGAGAAT (SEQ ID NO: 20).
- a 1088 bp fragment containing the *Mos1* transposase coding sequence amplified by PCR from pBluescribe M 13+/*Mos*1 using oJL77 5'-GCACGCGTTATGTCGAGTTTCGTGCCGAATAAAG (SEQ ID NO: 21) and oJL78 5'-GCGCTAGCTATTCAAAGTATTTGCCGTCGCTCGCGACACATTTTTCC CA (SEQ ID NO: 22). An artificial intron 5'-GTAAGTTTAAACATATATACTAACTAACCCATGGATTATTTAAATTT TCAG-3' (SEQ ID NO: 18) was inserted at position 264 with respect to the ATG.
- a 300 bp fragment containing the *glh-2* 3'UTR (nt 3287 to 4087 with respect to *glh-2* start codon) recovered form a 6.3 kb *glh-2* genomic fragment subcloned in pBluescript KS (Stratagene) (M. E. Gruidl et al., Proc. Natl. Acad. Sci. USA 93:13837-13842 (1996); gift of Karen Bennett, University of Missouri).

The transgenic strain EG1643 that contains the *Mos1* transposase expression vector has been generated by coinjection of *lin-15(n765)* wonns with:
- the Hind III-EcoRI fragment of pJL44 (injection concentration: 10 ng/µl)
- the Eag I-Kpn I *lin-15* genomic rescuing fragment from EKL15, S. L. McIntire et al., Nature 389:870-876 (1997)
- the Pst I-BsiW I fragment of pPD97/98 that drives expression of the Green Fluorescent Protein in the coelomocytes (gift of Piali Sengupta, Brandeis University) (injection concentration: 10 ng/µl)
- EcoRV-digested genomic DNA prepared from N2 worms (injection concentration: 80 ng/µl)

The resulting strain *lin-15(ts); oxEx166[hsp::MosTase:.glh-2; pPD97198; lin-15(+)]* has a wild-type phenotype. The presence of the extrachromosomal array causes expression of GFP in the coelomocytes which can be visualized using fluorescence microcopy.

### Mobilization of the transposon in the C. elegans genome

Mobilization of *Mos1* was achieved by crossing the transposase-expressing strain into worms containing the *Mos1* transposon-containing array. *lin-15(ts); oxEx166[hsp::MosTase:.-glh-2; pPD97198; lin-15(+)]* hermaphrodites were crossed with N2 males at 25°C. Non-Muv males *lin-15(ts); oxEx166* were crossed with *lin-15(ts); oxEx164[Mos1; rol-6(sd)]* Rol non-Muv hermaphrodites previously grown at 15°C.

The cross was kept at 20°C. Late L4 larvae or young adult Rol worms were transferred to a fresh plate and heat-shocked for 1 hour at 35°C. After 6 hours, non-Muv Rol PO animals (*lin-15(ts); oxEx164; oxEx166*) were transferred to a fresh plate and allowed to lay eggs for 48 hours. A fraction of the F1 animals contain insertions of *Mos1* in their genome and can be screened for mutant phenotypes.

### Identification of transposon insertion sites

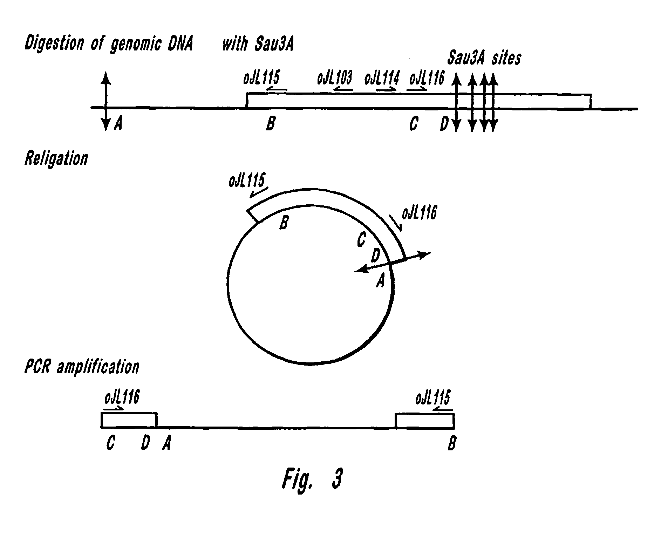

*Mos1* insertions were identified by inverse PCR, as shown in Figure 3. Genomic DNA was prepared according to standard procedure. Approximately 100 ng of genomic DNA was digested by Sau3A in a 10 µl volume for 3 to 14 hours. The restriction enzyme was inactivated by heating for 20 minutes at 70°C. Fragments were circularized by self-ligation (overnight incubation at 15°C with 5 units of T4 DNA ligase).

3 µl of ligated DNA was used for PCR amplification. A first round of amplification was performed using the primers oJL103 5'-TCTGCGAGTTGTTTTTGCGTTTGAG (SEQ ID NO: 3) and oJL114 5'-AAAGATTCAGAAGGTCGGTAGATGGG (SEQ ID NO: 10) (30 cycles, 45 seconds at 94°C / 1 minute at 60°C / 1 minute 15 seconds at 72°C, magnesium chloride concentration: 1.5 mM). The product of the first amplification was diluted 100-fold and subjected to a second round of amplification using the nested primers oJL115 5'-GCTCAATTCGCGCCAAACTATG (SEQ ID NO: 11) and oJL116 5'-GAACGAGAGGCAGATGGAGAGG (SEQ ID NO: 12) (25 cycles, 45 seconds at 94°C / 1 minute at 62°C / 1 minute 15 seconds at 72°C, magnesium chloride concentration: 2.5 mM). Resulting fragments were run on an agarose gel, gel-purified and sequenced either directly or after subcloning.

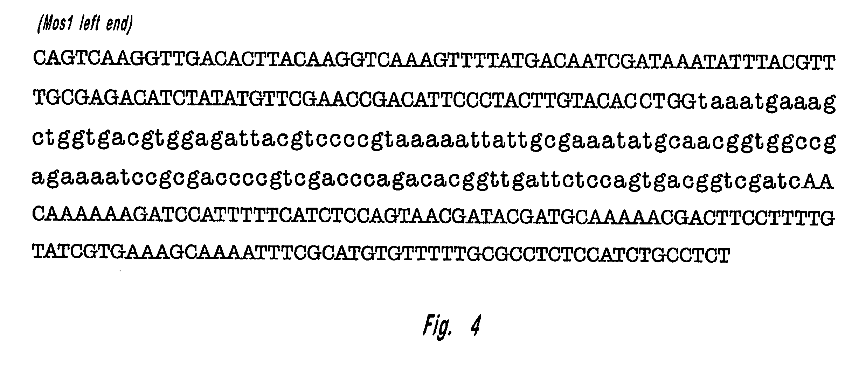

Figure 4 contains the sequence of an inverse PCR product demonstrating insertion of *Mos1* in chromosome X. Nucleotides in capital letters are from the *Mos1* transposon. *C*. *elegans*-flanking genomic region is in lower case. It matches the Y47C4.Contig215 sequence from chromosome X available at the Sanger Centre.

*Mos1*, a mariner-*like* transposon isolated from *Drosophila mauritiana* was used. Transgenic worms containing *Mos1* in an extrachromosomal array were crossed with transgenic worms containing an expression vector in which a heat-shock promoter (*hsp 16-48*) drives the expression of the *mos* transposase (Figure 1). Cross-progeny containing both the *Mos1* transposon and the *mos* transposase were isolated. Heat-shock of these worms induced the expression of the transposase which in turn caused *Mos1* elements to transpose from the extrachromosomal array into the *C*. *elegans* genome. Five insertions were isolated, for a rate of one in seventeen animals analyzed. However, this array is only 20% stable per generation. Thus, therein on average one transposition into chromosomes for every three germ cells exposed to the transposon.

Some insertions will disrupt genes and cause mutant phenotypes. Mutant worms are outcrossed with wild-type worms containing no *Mos1* transposon. Since the insertion responsible for the mutation cosegregates with the mutant phenotype, it is possible to isolate the single relevant *Mos1* insertion after only a few outcrosses. Genomic DNA is then prepared from the outcrossed mutant. Regions flanking the transposon are recovered by inverse PCR and sequenced. Comparison of flanking sequences with the *C*. *elegans* genome sequence allows immediate identification of the mutated gene. This new mutagenesis system will significantly speed up the identification of genes of interest using *C*. *elegans* as a genetic model.

### Example 9 - Mos1 Mutagenesis and Rapid Cloning of Genes

In one embodiment, the method described in this invention is capable of generating mutations which can be rapidly cloned based on the *Mos1* unique DNA tag. To demonstrate that this is true, mutants have been identified and the relevant genes have been cloned using inverse PCR. Specifically, a morphological mutant in *C*. *elegans* was isolated which causes the worms to be short and squat. Such mutations are called dumpy mutations and are given the three letter designation "dpy." A dumpy animal was identified after mobilization of the wild-type *Mos1* transposon. DNA was prepared, cleaved with the restriction enzyme Sau3A, and religated. Inverse PCR was performed using primers contained within the transposon but facing outward. The amplified fragment was sequenced. The *Mos1* element was inserted 175 nucleotides 5' of F54D8.1, which encodes a collagen protein. An inspection of the genetic map demonstrated that this insertion is in a chromosomal interval which also contains the *dpy-17* gene which had been previously defined by point mutations using chemical mutagens by Sydney Brenner in 1974. A complementation test was performed and the test demonstrated that this mutation was an allele of *Dpy-17.* Thus, the method is capable of rapidly demonstrating the molecular identity of a gene which had remained unknown for almost 30 years. Mutants incapable of detecting high osmotic gradients (Osm) were also screened for. The first Osm mutant identified was cloned in a similar manner and proved to be an insertion of *Mos1* in exon 10 of the *eat-4* gene.

### Example 10 - Targets of Transposase and Transposon

For *Mos1* insertions to be useful for the cloning of mutated genes, the transposase must specifically mobilize *Mos1* and not other mariner elements. The *C*. *elegans* genome contains endogenous transposons. Apart from the most active Tc1 and Tc3 transposons, which are distantly related to *Mos1,* every haploid genome contains at least 55 copies of a Mariner Like Element (MLE), which is closely related to *Mos1.* M. M. Sedensky, S. J. Hudson, B. Everson, P. G. Morgan, Nucleic Acids Res. 22, 1719 (1994); H. M. Robertson, D. J. Lampe, Mol. Biol. Evol. 12, 850 (1995). Since in a few cases transposases of the Mariner family have been shown to cross-mobilize distinct but related transposons (P. Sundararajan, P. W. Atkinson, D. A. O'Brochta, Insect Mol. Biol. 8, 359 (1999)), it was tested whether *Mos* transposase expression had triggered transposition of the endogenous MLEs. Eight strains in which *Mos1* insertions had occurred were analyzed by Southern blot for changes in MLE distribution. No changes in MLE distribution were detected. Worm genomic DNA of *lin-15(n765)* and *Mos1*-containing strains was extracted, Bgl II digested and run for Southern blot analysis using standard procedures. Oligos oJL132: 5'-ATATGCGGTGCGATGGGTGAG (SEQ ID NO: 8) and oJL133: 5'-GGCGAACGCGATGAGAAGAAAG (SEQ ID NO: 9) were used to amplify a 842 bp MLE fragment from N2 worm genomic DNA. The PCR product was sequenced and used for probe synthesis (data not shown), indicating that *Mos* transposase is specific for *Mos1* in the *C*. *elegans* germline.

How many insertions occurred in every animal and what were their distributions? The number of chromosomal insertions per strain was determined by Southern blot analysis in eight insertion strains. Only one insertion per strain was detected (Figure 7A). To determine the location of the mobilized transposons, the left junctions of 17 insertions were cloned using inverse PCR. Approximately 100 ng of total genomic DNA was digested with Sau3A, self-ligated under dilute conditions, and then 3% of the ligation was subjected to two rounds of nested PCR using the following primers: oJL103 (SEQ ID NO: 3)/oJL114 5'-AAAGATTCAGAAGGTCGGTAGATGGG (SEQ ID NO: 10) (first PCR), oJL115 5'-GCTCAATTCGCGCCAAACTATG (SEQ ID NO: II) / oJL116 5'-GAACGAGAGGCAGATGGAGAGG (SEQ ID NO: 12) (second PCR). PCR products were purified on agarose gel and sequenced using oJL115 (SEQ ID NO: 11) as a primer. In agreement with the Southern blot experiments, only one insertion per strain was detected. Insertion sites were distributed on all six chromosome (Figure 7B). Transposition occurred into exons, introns and intergenic regions (Table 2). Sequences flanking both sides of the transposon were determined for nine of the localized insertions. In each case, the inverted terminal repeats were complete and flanked by a TA dinucleotide that arose from the duplication of the original TA found in the genomic sequence (Figure 7C).

Table 2. Properties of *Mos1* genomic insertions. *Mos1* flanking were compared with the *C*. *elegans* genome sequence. Physical location in the genome is given as the nucleotide position of the corresponding clone in the *C*. *elegans* database (ACeDB).

| Isolation Name | Transposon source | Physical location | Interpolated genetic location | | Genefinder predictions |
|---|---|---|---|---|---|
| oxTi1 | Extrachromosomal | Y65B4BL @ 27,362 | LGI, | -19 | Intergenic |
| oxTi2 | Extrachromosomal | Y44E3A @ 34,440 | LGI, | -4.75 | Intergenic |
| oxTi3 | Extrachromosomal | M01E5 @ 19,740 | LGI, | +29.9 | Intergenic |
| oxTi4 | Extrachromosomal | T13C2 @ 4,948 | LGII, | +0.1 | Exon #4 of F41G.12 |
| oxTi5 | Extrachromosomal | K08E5 @ 31,631 | LGIII, | +4.61 | Intergenic |
| oxTi6 | Extrachromosomal | H23L24 @ 4,529 | LGIV, | +3.9 | Intergenic |
| oxTi7 | Extrachromosomal | K08D8 @ 4,234 | LGIV, | +6.6 | Intergenic |
| oxTi8 | Extrachromosomal | R09B5 @ 22,929 | LGV, | -19.0 | Exon #6 of R09B5.12 |
| oxTi9 | Extrachromosomal | Y69H2 @ 39,771 | LGV, | +17.49 | Intron #5 of Y69H2.4 |
| oxTi10 | Extrachromosomal | Y47C4A | LGX, | -20 | Repeat |
| oxTi11 | Extrachromosomal | C34E11 @ 12,022 | LGX, | +6.55 | Exon #10 of C34E11.1 |
| oxTi12 | Integrated array | Y71A12B @ 50,370 | LGI, | +21 | Intergenic |
| oxTi13 | Integrated array | Y48G1C @ 19,916 | LGI, | -19.8 | Intergenic |
| oxTi14 | Integrated array | C17F4 @ 22,793 | LGII, | -8.06 | Exon #18 of *gcy-19* |
| oxTi15 | Integrated array | F35C5 @ 5,735 | LGII, | +10 | Intergenic |
| oxTi16 | Integrated array | C06B3 @ 14,747 | LGV, | +5.79 | Intergenic |
| oxTi17 | Integrated array | R01H2 @ 20,193 | LGIII, | -0.86 | Intergenic |

Comparison of the insertion site sequences did not reveal a strong consensus motif for the target DNA. Molecular analysis of the insertions therefore demonstrated that *Mos1* insertion obeyed properties previously observed for mariner class transposons. However, a formal possibility remained that *Mos1* hopped into the genomic DNA present in one of the extrachromosomal arrays and that recombination occurred subsequently between the array and the genome. To rule out this possibility, the insertion *oxTi4* which was positioned 35 kb away from *snt-1* was genetically mapped. In agreement with this physical location, *oxTi4* was mapped less than 2.5 map units from *snt-1:* 20 Snt-1 individuals were cloned from the self-progeny of *oxTi4*/*snt-1* hermaphrodites. None of the mutants segregated *oxTi4.* The presence of *oxTi4* was determined by PCR using one *Mos1* primer pointing towards the right end of the transposon (oJL89 (SEQ ID NO: 5)) and one primer in the genome (oJL129 5'-CCAAATGCGTCTGTCCCACTC (SEQ ID NO: 13)). A PCR positive control was performed on each DNA sample using *cha-1* primers.

### Example 11 - Remobilization of a Genomic Transposon Insertion

The transposition events documented above were all excisions from an array of transposons residing in *Drosophila* DNA. To determine whether the transposase acts on a single *Mos1* transposon in a *C*. *elegans* chromosome, the *oxTi4* insert was remobilized. Primers for PCR were designed flanking the *oxTi4* insertion. A first PCR round was performed with primers located 1671 nt upstream and 3144 bp downstream to *oxTi4* (respectively oJL149 5'-AAGTATGGCCAAACGACCCGACAC (SEQ ID NO: 14) and oJL150 5'-GCATTGGCACCTTTCTCCCTTCT (SEQ ID NO: 15)). A second round was performed using primers 493 bp upstream and 913 downstream to *oxTi4* (respectively oJL 145 5'-ACAGGCAGCATTTTGTAGTCT (SEQ ID NO: 16) and oJL148 5'-AGGCTGCCTCGTAAGTTCCTACAG (SEQ ID NO: 17)). Short PCR products were gel purified, subcloned and sequenced. The transposase-expressing transgene (*oxEx167(glh-2:Transposase]*) was crossed into animals homozygous for the *oxTi4* insertion and DNAs from the progeny were analyzed for amplified fragments shorter than the insertion. These shorter PCR products represented a variety of excision events, including the three nucleotide excision footprint previously characterized for *Mos1* excisions (G. Bryan, D. Garza, D. Hartl, *Genetics* **125**, 103 (1990)), as well as smaller footprints, excisions and even incomplete excisions (Table 3). Since these products could arise from excision events in somatic cells, progeny animals that lost the transposase expression array were analyzed. Pools of 15 individuals from *oxTi4; oxEa167[glh-2:Transposase]* progeny that lost the transposase array were transferred to fresh plates and allowed to lay eggs for 24 hours. Adult worms were then analyzed by a single round of PCR using the primers oJL145 (SEQ ID NO: 16)-oJL148 (SEQ ID NO: 17). Sixty individuals were cloned from the progeny of the pool exhibiting short PCR product and analyzed at the next generation to identify clones that lost *oxTi4.* One animal was identified among 954 progeny in which excision of the transposon had occurred. In this animal the excision left a 3 bp footprint and the duplicated TA dinucleotide which together resulted in a +2 frameshift. These data indicate that single copies of the *Mos1 Drosophila* transposon can excise from *C*. *elegans* DNA in the germline to introduce frameshift or deletion mutations at the transposon insertion site.

Table 3. Lesions generated by excision of the *oxTi4* insert. The extrachromosomal [*glh-2 : Transposase*} transgene was crossed into animals homozygous for the *oxTi4* insertion. PCR was used to analyze the *oxTi4* insertion site after the loss *of Mos 1*. Pools of 15 individuals from *oxTi4; oxExl67* [*glh-2 : Transposase*] progeny that lost the transposase array were transferred to fresh plates and allowed to lay eggs for 24 hours. Adult worms were then analyzed by a single round of PCR using the primers oJL145 (SEQ ID NO: 16)-oJL148 (SEQ ID NO: 17). Sixty individuals were cloned from the progeny of the pool exhibiting short PCR product and analyze at the next generation to identify clones that lost *oxTi4.* Top line: sequence of *oxTi4.* Lower case: *Mos 1* sequence. Upper case: genomic sequence. Bold: TA dinucleotide duplicated during *Mos 1* insertion. Bottom lines: excision products. Dash: deleted base pairs. The insertion (bottom line, italic letters) corresponds to an internal fragment of *Mos1* (nt 147 to 178).

| |
|---|
| CTCTTTTCCAGACGAGTAccaggtgtac........... tacacctgaTATATCCTTTTGTTCCTT |
| CTCTTTTCCAGACGAGTA--------------------------------- TATATCCTTTTGTTCCTT |
| CTCTTTTCCAGACGAGTA--------------------------------- aTATATCCTTTTGTTCCTT |
| CTCTTTTCCAGACGAGTA------------------------------- tgaTATATCCTTTTIGTTCCTT |
| CTCTTTTCCAGACGAGTAc--------------------------------- TATATCCTTTTGTTCCTT |
| ---------------------------249 bp deletion----tgaTATATCCTTTTGTTCCTT |
| CTCTTTTCCAGACGAGa----------143 bp deletion------------------------ |
| CTCTTTTCCAGACGAGTA---------188 bp deletion------------------------ |
| ---------------------------463 bp deletion--------- |
| CTCTTTTCCAGACGAGTA attgtttactctcagtgcagtcaacatgtcgaTATCCTTTTGTTCCTT |

### Example 12: - Engineering Mutations in the C. elegans Genome by Transgene Instructed DNA Double Strand Break Repair Following Mos1 Excision

Germline expression of the *Mos* transposase under the control of the *glh-2* promoter causes reexcision of single copies of *Mos1* inserted in the *C*. *elegans* genome. Remobilization of the transposon causes a DNA double strand break (DSB) at the site of excision which is repaired by the cellular machinery. In 1992, R. Plasterk and J. Groenen (EMBO J. 11:287) demonstrated that a DSB caused by excision of a Tc1 transposon in a *mut-6(st702)* background can be repaired using DNA contained in an extrachromosomal array that carries sequences homologous to the region of excision. As a result, sequences flanking the break can be replaced by sequences contained in the transgene. This strategy provides a way to engineer mutations in the genome. However, this approach never became a routine strategy probably because transposition is not controlled and excision occurs at low rates in such mutant strains.

The controlled transposition of *Mos1* provides an efficient tool to use this strategy for engineering of the *C*. *elegans* genome: after a *Mos1* insertion has been identified in the gene of interest, a transgene is constructed with mutated sequences homologous to the region of insertion. The transgene that carries the *glh-2::Mos* transposase expression vector is crossed into the strain that contain the *Mos1* genomic insertion and the template transgene. Expression of *Mos* transposase causes *Mos1* excision and the progeny is screened by PCR for transgene instructed repair at the excision site (Figure 8).

The feasibility of regulated mobilization of a heterologous transposon in the *C. elegans* germline was thus demonstrated. The characteristics of *Mos1* transposition suggest that it could be used as a technique for tagging mutant genes. First, the *Mos* transposase does not activate transposition of endogenous transposons. Second, transposition *of Mos* in the germline is strictly dependent on the expression of the transposase. In this respect, the use of a heat-shock promoter to express the transposase is if particular interest since it provides a convenient way to turn transposition on and off and to stabilize new inserts. Third, insertion sites of *Mos1* in the genome do not exhibit strong sequence bias. Transposons were inserted into exons, introns and intergenic regions. Comparison of the insertion sites did not reveal a strong consensus sequence apart from the TA dinucleotide. Fourth, excision and insertion frequencies can be differentially manipulated by expressing the transposase under the control of different promoters. The heat-shock promoter caused very low rates of excision and loss of the transposon array but high rates of transposon insertion. The *glh-2* promoter construct caused a low rate of insertion but a high rate of excision and loss of the transposon array. Since transposon insertions frequently do not disrupt gene function in *C*. *elegans* even if the insertion occurs in an exon (A. M. Rushforth, B. Saari, P. Anderson, Mol. Cell. Biol. 13, 902 (1993); A. M. Rushforth, P. Anderson, Mol. Cell. Biol. 16, 422 (1996)), transposons are usually remobilized to generate deletion alleles (D. Eide, P. Anderson, Mol. Cell. Biol. 8, 737 (1988); R. R. Zwaal, A. Broeks, J. van Meurs, J. T. Groenen, R. H. Plasterk, Proc. Natl. Acad. Sci. USA 90, 7431 (1993)). It was thus demonstrated that the *glh-2* expression construct can be used to generate deletion alleles of the genes containing *Mos1* insertions.

*Mos1* transposition in *C*. *elegans* will allow the development of two new genetic tools. First, mutations identified in forward screens using *Mos1* will allow the rapid cloning of the mutated gene. Second, a library of insertions localized in the genome could be generated; the *glh-2* expression construct could then be used to remobilize these insertions at high frequency and generate deletion and frameshift mutations in genes of interest.

## Claims

1. A method of regulated expression of a heterologous transposase gene in cells of the germline of *C*. *elegans* comprising the steps of:
a. inserting a transgene construct into the *C*. *elegans,* wherein the construct comprises the heterologous transposase gene operably linked to a regulable expression control element and a 3' untranslated region of a gene that is expressed in *the C. elegans* germline, wherein the 3' untranslated region comprises a *glh-2*3' untranslated region; and
b. expressing the heterologous transposase gene.

2. The method of Claim 1, wherein the transposase gene is a TC3A transposase gene.

3. The method of any preceding claim, wherein the regulable expression control element is an inducible promotor.

4. The method of any preceding claim, wherein the regulable expression control element comprises a heat-shock promotor.

## Patentansprüche

1. Verfahren zur regulierten Expression eines heterologen Transposase-Gens in Zellen der Keimbahn von *C. elegans,* welches die Schritte umfasst:
a. Einführen eines Transgen-Konstrukts in *C. elegans,* wobei das Konstrukt das heterologe Transposase-Gen umfasst, funktionsfähig verknüpft mit einem regulierbaren Expressionskontrollelement und einer 3'-nichttranslatierten Region eines Gens, das in der Keimbahn von *C. elegans* exprimiert wird, wobei die 3'-nicht-translatierte Region eine *glh-2* 3'-nicht-translatierte Region umfasst; und
b. Exprimieren des heterologen Transposase-Gens.

2. Verfahren gemäß Anspruch 1, wobei das Transposase-Gen ein TC3A Transposase-Gen ist.

3. Verfahren gemäß einem der vorhergehenden Ansprüche, wobei das regulierbare Expressionskontrollelement ein induzierbarer Promotor ist.

4. Verfahren gemäß einem der vorhergehenden Ansprüche, wobei das regulierbare Expressionskontrollelement einen Heat-Shock Promotor umfasst.

## Revendications

1. Procédé d'expression régulée d'un gène de transposase hétérologue dans des cellules de la lignée germinale de *C.elegans* comprenant les étapes:
a. insérer un constituant de transgène dans le *C.elegans,* dans lequel le constituant comprend le gène de transposase hétérologue opérationnellement lié à un élément du contrôle de l'expression pouvant être régulé et une zone non traduite 3' d'un gène, qui est exprimé dans la lignée germinale de *C.elegans,* dans lequel la zone non traduite 3' comprend une zone non traduite *glh-2* 3'; et
b. exprimer le gène de transposase hétérologue.

2. Procédé selon la revendication 1, dans lequel le gène de transposase est un gène de transposase TC3A.

3. Procédé selon l'une quelconque des revendications précédentes, dans lequel l'élément de contrôle de l'expression pouvant être régulé est un promoteur inductible.

4. Procédé selon l'une quelconque des revendications précédentes, dans lequel l'élément de contrôle de l'expression pouvant être régulé comprend un promoteur de choc thermique.


### SEQUENCE LISTING

<110> university of Utah Research Foundation
   Jorgensen, Erik M.
   Bessereau, Jean-Louis
<120> Method of Transposon-Mediated Mutagenesis in the Nematode caenorhabditis Elegans
<130> 1321.2.28
<150> 60/136,972
   <151> 1999-06-01
<160> 23
<170> PatentIn version 3.0
<210> 1
   <211> 26
   <212> DNA
   <213> Artificial Sequence
<220>
   <223> Synthetic Oligonucleotide
<400> 1
   caaccttgac tgtcgaacca ccatag 26
<210> 2
   <211> 23
   <212> DNA
   <213> Artificial Sequence
<220>
   <223> Synthetic oligonucleotide
<400> 2
   acaaagagcg aacgcagacg agt 23
<210> 3
   <211> 25
   <212> DNA
   <213> Artificial Sequence
<220>
   <223> Synthetic Oligonucleotide
<400> 3
   tctgcgagtt gtttttgcgt ttgag 25
<210> 4
   <211> 18
   <212> DNA
   <213> Artificial Sequence
<220>
   <223> Synthetic Oligonucleotide
<400> 4
   cgcatgcggc ttactcac 18
<210> 5
   <211> 22
   <212> DNA
   <213> Artificial Sequence
<220>
   <223> Synthetic Oligonucleotide
<400> 5
   ggccccatcc gattaccacc ta 22
<210> 6
   <211> 21
   <212> DNA
   <213> Artificial Sequence
<220>
   <223> Synthetic Oligonucleotide
<400> 6
   gcgaaacgca taccaactgt a 21
<210> 7
   <211> 21
   <212> DNA
   <213> Artificial Sequence
<220>
   <223> Synthetic Oligonucleotide
<400> 7
   ttcatgccga aaagcaggcg t 21
<210> 8
   <211> 21
   <212> DNA
   <213> Artificial Sequence
<220>
   <223> Synthetic Oligonucleotide
<400> 8
   atatgcggtg cgatgggtga g 21
<210> 9
   <211> 22
   <212> DNA
   <213> Artificial Sequence
<220>
   <223> Synthetic Oligonucleotide
<400> 9
   ggcgaacgcg atgagaagaa ag 22
<210> 10
   <211> 26
   <212> DNA
   <213> Artificial Sequence
<220>
   <223> Synthetic Oligonucleotide
<400> 10
   aaagattcag aaggtcggta gatggg 26
<210> 11
   <211> 22
   <212> DNA
   <213> Artificial Sequence
<220>
   <223> Synthetic Oligonucleotide
<400> 11
   gctcaattcg cgccaaacta tg 22
<210> 12
   <211> 22
   <212> DNA
   <213> Artificial Sequence
<220>
   <223> Synthetic Oligonucleotide
<400> 12
   gaacgagagg cagatggaga gg 22
<210> 13
   <211> 21
   <212> DNA
   <213> Artificial Sequence
<220>
   <223> Synthetic Oligonucleotide
<400> 13
   ccaaatgcgt ctgtcccact c 21
<210> 14
   <211> 24
   <212> DNA
   <213> Artificial Sequence
<220>
   <223> Synthetic Oligonucleotide
<400> 14
   aagtatggcc aaacgacccg acac 24
<210> 15
   <211> 23
   <212> DNA
   <213> Artificial Sequence
<220>
   <223> Synthetic Oligonucleotide
<400> 15
   gcattggcac ctttctccct tct 23
<210> 16
   <211> 21
   <212> DNA
   <213> Artificial Sequence
<220>
   <223> Synthetic Oligonucleotide
<400> 16
   acaggcagca ttttgtagtc t 21
<210> 17
   <211> 24
   <212> DNA
   <213> Artificial Sequence
<220>
   <223> Synthetic Oligonucleotide
<400> 17
   aggctgcctc gtaagttcct acag 24
<210> 18
   <211> 51
   <212> DNA
   <213> Artificial Sequence
<220>
   <223> Synthetic Oligonucleotide
<400> 18
   gtaagtttaa acatatatac taactaaccc atggattatt taaattttca g 51
<210> 19
   <211> 26
   <212> DNA
   <213> Artificial Sequence
<220>
   <223> Synthetic Oligonucleotide
<400> 19
   cgaagcttgc tggacggaaa tagtgg 26
<210> 20
   <211> 26
   <212> DNA
   <213> Artificial Sequence
<220>
   <223> Synthetic Oligonucleotide
<400> 20
   cgacgcgttc ttgaagttta gagaat 26
<210> 21
   <211> 34
   <212> DNA
   <213> Artificial Sequence
<220>
   <223> Synthetic Oligonucleotide
<400> 21
   gcacgcgtta tgtcgagttt cgtgccgaat aaag 34
<210> 22
   <211> 49
   <212> DNA
   <213> Artificial Sequence
<220>
   <223> Synthetic Oligonucleotide
<400> 22
   gcgctagcta ttcaaagtat ttgccgtcgc tcgcgacaca tttttccca 49
<210> 23
   <211> 347
   <212> DNA
   <213> Artificial Sequence
<220>
   <223> Inserted transposon and flanking regions
<400> 23
   cagtcaaggt tgacacttac aaggtcaaag ttttatgaca atcgataaat atttacgttt    60
   gcgagacatc tatatgttcg aaccgacatt ccctacttgt acacctggta aatgaaagct   120
   ggtgacgtgg agattacgtc cccgtaaaaa ttattgcgaa atatgcaacg gtggccgaga   180
   aaatccgcga ccccgtcgac ccagacacgg ttgattctcc agtgacggtc gatcaacaaa   240
   aaagatccat ttttcatctc cagtaacgat acgatgcaaa aacgacttcc ttttgtatcg   300
   tgaaagcaaa atttcgcatg tgtttttgcg cctctccatc tgcctct                 347